# EUROPÄISCHE PATENTSCHRIFT

(11) **EP 3 980 497 B1**
(45) Veröffentlichungstag und Bekanntmachung des Hinweises auf die Patenterteilung: **23.10.2024**
(21) Anmeldenummer: 20734318.7
(22) Anmeldetag: 29.05.2020
(51) Int. Cl.: C09B 33/22, C09D 11/17, C09D 11/328

(54) **TRISAZOVERBINDUNGEN FÜR DEN TINTENSTRAHLDRUCK**
TRISAZO COMPOUNDS FOR INK-JET PRINTING
COMPOSÉS TRISAZOÏQUES POUR L'IMPRESSION PAR JET D'ENCRE

(30) Priorität: 06.06.2019 EP 19178667
(43) Veröffentlichungstag der Anmeldung: 13.04.2022
(73) Patentinhaber: LANXESS Deutschland GmbH, 50569 Köln (DE)
(72) Erfinder: LIMPER, Dominik, 51379 Leverkusen (DE); MOIGNO, Damien, 1723 Marly (CH)
(86) Internationale Anmeldenummer: PCT/EP2020/065033
(87) Internationale Veröffentlichungsnummer: WO 2020/245052

(56) Entgegenhaltungen:
- WO-A1-2016/078988

## Beschreibung

Die vorliegende Erfindung betrifft neue Trisazoverbindungen und deren Salze, Verfahren zu deren Herstellung und ihre Verwendung zum Färben und Bedrucken von natürlichen und synthetischen Materialien, insbesondere zum Einsatz für den Tintenstrahldruck.

Aus dem Stand der Technik sind bereits eine Reihe von schwarzen Farbstoffen für die Anwendung in Tintenstrahldruckverfahren bekannt.

Im Bereich des industriellen Tintenstrahldrucks werden beispielsweise die Farbstoffe C.I. Acid Black 1 oder C.I. Direct Black 19 häufig eingesetzt.

Weiterhin sind aus der EP-A 3 020 770 schwarze Trisazofarbstoffe für den Einsatz in Tintenstrahldruckverfahren bekannt.

Die aus dem Stand der Technik bekannten Farbstoffe sind jedoch in mancher Hinsicht noch verbesserungswürdig. Insbesondere besitzen die aus dem Stand der Technik bekannten Farbstoffe keine ausreichende Lagerstabilität was sich in einer Veränderung sowohl der Farbstärke als auch des Farbortes bei Lagerung der flüssigen Farbstofflösungen bemerkbar macht.

Es besteht daher weiterhin Bedarf an neuen Farbstoffen für den Tintenstrahldruck, die die oben genannten Nachteile überwinden.

Aufgabe der vorliegenden Erfindung ist daher die Bereitstellung neuer Farbstoffe zum Färben und Bedrucken von natürlichen und synthetischen Materialien in schwarzen Farbtönen, insbesondere zur Anwendung für den Tintenstrahldruck.

Gegenstand der vorliegenden Erfindung sind Trisazoverbindungen der Formel (I) 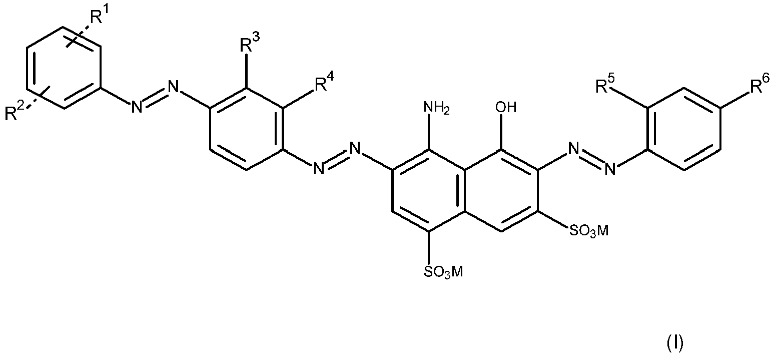 worin
R¹ für SO₃M oder COOM steht, und
R² für SO₃M oder COOM steht,
R³ und R⁴ unabhängig voneinander für Wasserstoff oder CH₃ stehen,
R⁵ und R⁶ unabhängig voneinander für SO₃M, COOM oder NO₂ stehen, und
M für Wasserstoff, ein einwertiges Metallkation, für Ammonium oder für Alkylammonium, welches ein- oder mehrfach, gleich oder verschieden durch C₁-C₄-Alkyl substituiert ist, steht.

Bevorzugt sind Trisazoverbindungen der Formel (I), worin einer der Reste R⁵ oder R⁶ für NO₂ steht, Als einwertige Metallkationen in der Bedeutung von M kommen beispielsweise Natrium-, Kalium-, oder Lithium-Ionen in Frage.

Als Alkylammonium welches ein- oder mehrfach, gleich oder verschieden durch C₁-C₄-Alkyl substituiert ist kommen beispielsweise Trimethylammonium, Triethylammonium, Triisopropylammonium, Tributylammonium, in Frage, vorzugsweise Triethylammonium und Triisopropylammonium.

Die Trisazoverbindungen der allgemeinen Formel (I) können als freie Säure oder in Form von anorganischen oder organischen Salzen. Bevorzugt liegen sie als Alkali- oder Ammoniumsalz, insbesondere als Natriumsalze vor.

M in den Bedeutungen SO₃M und COOM der Reste R¹, R², R³, R⁵ und R⁶ kann gleich oder verschieden sein. Vorzugsweise hat M in den Bedeutungen SO₃M und COOM der Resten R¹, R², R³, R⁵ und R⁶ jeweils die gleiche Bedeutung.

Bevorzugt sind Trisazoverbindungen der Formel (I) worin
R¹ für SO₃M oder COOM steht, und
R² für SO₃M oder COOM steht.
R³ und R⁴ unabhängig voneinander für Wasserstoff oder CH₃ stehen, und
R⁵ und R⁶ unabhängig voneinander für SO₃M, COOM oder NO₂ stehen, mit der Maßgabe, dass einer der Reste R⁵ oder R⁶ für NO₂ steht, und
M für Wasserstoff, Natrium, Kalium, Lithium, Ammonium, oder Alkylammonium, welches ein- oder mehrfach, gleich oder verschieden durch C₁-C₂-Alkyl substituiert ist, steht.

Besonders bevorzugt sind Trisazoverbindungen der Formel (I), worin
R¹ für SO₃M oder COOM steht, und
R² für SO₃M oder COOM steht.
R³ und R⁴ unabhängig voneinander für Wasserstoff oder CH₃ stehen, und
R⁵ und R⁶ unabhängig voneinander für SO₃M, COOM oder NO₂ stehen,
mit der Maßgabe, dass einer der Reste R⁵ oder R⁶ für NO₂ steht, und
M für Wasserstoff, Natrium, Kalium, Lithium, Ammonium, Trimethylammonium oder Triethylammonium steht.

Ganz besonders bevorzugt sind Trisazoverbindungen der Formel (I), worin
R¹ für SO₃M oder COOM steht, und
R² für SO₃M oder COOM steht.
R³ und R⁴ für Wasserstoff oder CH₃ stehen,
mit der Maßgabe, dass R³ und R⁴ nicht beide gleichzeitig für Wasserstoff und nicht beide gleichzeitig für CH₃ stehen,
R⁵ und R⁶ für SO₃M, COOM oder NO₂ stehen,
mit der Maßgabe, dass einer der Reste R⁵ oder R⁶ für NO₂ steht,
   und
M für Wasserstoff, Natrium, Kalium, Lithium, Ammonium, Trimethylammonium oder Triethylammonium steht.

Insbesondere bevorzugt sind solche Verbindungen der Formel (I), die den Formeln (la) oder (Ib) 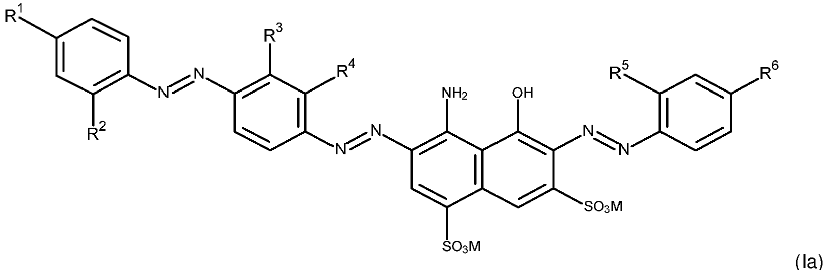 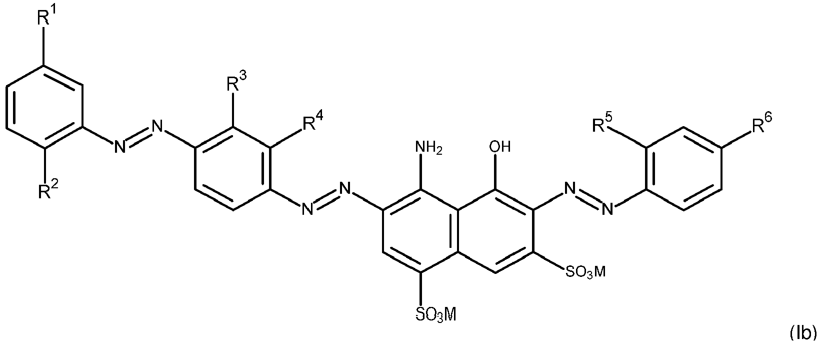 entsprechen,
worin
R¹ für SO₃M oder COOM steht, und
R² für SO₃M oder COOM steht,
R³ und R⁴ für Wasserstoff oder CH₃ stehen,
mit der Maßgabe, dass R³ und R⁴ nicht beide gleichzeitig für Wasserstoff und nicht beide gleichzeitig für CH₃ stehen,
R⁵ und R⁶ für SO₃M, COOM oder NO₂ stehen,
mit der Maßgabe, dass einer der Reste R⁵ oder R⁶ für NO₂ steht,
   und
M für Wasserstoff, Natrium, Kalium, Lithium, Ammonium, Trimethylammonium oder Triethylammonium steht

Insbesondere bevorzugt sind Verbindungen der Formel (I), (la) und (Ib),
worin
R¹ für SO₃M steht,
R² für SO₃M steht,
R³ und R⁴ für Wasserstoff oder CH₃ stehen,
mit der Maßgabe, dass R³ und R⁴ nicht beide gleichzeitig für Wasserstoff stehen und nicht beide gleichzeitig für CH₃ stehen,
R⁵ und R⁶ für SO₃M, COOM oder NO₂ stehen,
mit der Maßgabe, dass einer der Reste R⁵ oder R⁶ für NO₂ steht,
   und
M für Wasserstoff, Natrium, Kalium, Lithium, Ammonium oder Triethylammonium steht.

Ebenfalls insbesondere bevorzugt sind Verbindungen der Formel (I), (la) und (Ib),
worin
R¹ für COOM steht,
R² für COOM steht,
R³ und R⁴ für Wasserstoff oder CH₃ stehen,
mit der Maßgabe, dass R³ und R⁴ nicht beide gleichzeitig für Wasserstoff stehen und nicht beide gleichzeitig für CH₃ stehen,
R⁵ und R⁶ für SO₃M, COOM oder NO₂ stehen,
mit der Maßgabe, dass einer der Reste R⁵ oder R⁶ für NO₂ steht,
   und
M für Wasserstoff, Natrium, Kalium, Lithium, Ammonium oder Triethylammonium steht.

Die erfindungsgemäßen Trisazoverbindungen der Formel (I) eignen sich hervorragend zum Färben und Bedrucken unterschiedlicher Materialien, insbesondere in schwarzen Farbtönen.

Die erfindungsgemäßen Trisazoverbindungen der Fomel (I) eignen sich insbesondere zum Färben und Bedrucken von cellulosehaltigen Materialien, insbesondere Papier, Baumwolle, Leinen und Viskose, von tierischen Häuten und Haaren, insbesondere Leder und Wolle, von Eierschalen sowie nanoporösen Materialien und Metallen..

Die erfindungsgemäßen Trisazoverbindungen können vorzugsweise zur Massen- oder Oberflächenfärbung von Papier eingesetzt werden. Die Farbstoffe können auch zum Färben von Garnen und Stückwaren aus Baumwolle, Viskose und Leinen im Ausziehverfahren aus einer langen Flotte oder im kontinuierlichen Verfahren verwendet werden.

Insbesondere eigenen sich die erfindungsgemäßen Trisazoverbindungen als Farbstoffe für wässrige und auf organischen Lösungsmitteln basierte Tinten, insbesondere als Aufzeichnungsflüssigkeiten für den Tintenstrahldruck (Ink-Jet Druck) sowie als Farbstoffe für Schreibvorrichtungen wie z.B Stifte und Stempel. Insbesondere eignen sie sich für den Tintenstrahldruck auf porösen, insbesondere auf nanoporösen Aufzeichnungsblättern sowie auf Metallen, Papier und anderen cellulosehaltien Materialien sowie Eierschalen.

Nanoporöse Aufzeichnungsblätter sind beispielsweise Blätter aus nanoporösen anorganischen Verbindungen wie zum Beispiel Siliziumdioxid, Aluminiumoxid/Hydroxid, Aluminiumoxid oder deren Mischungen.

Die erfindungsgemäßen Trisazoverbindungen eignen sich insbesondere als Färbemittel für die Herstellung von flüssigen Formulierungen für den Tintenstrahldruck und für Schreibvorrichtungen. oder bei der Herstellung von Farbfiltern für optische und optoelektonische Anwendungen.

Die dabei erhaltenen Färbungen und Drucke entsprechen den höchsten qualitativen Anforderungen. Überraschender Weise zeichnen sich die erfindungsgemäßen Trisazoverbindungen der Formel (I) dadurch aus, dass sie in wässriger Lösung eine deutlich verbesserte Lagerstabilität aufweisen. Hierdurch ist es möglich die wässrigen Lösungen der erfindungsgemäßen Trisazoverbindungen über längere Zeiträume zu lagern, ohne dass sich Farbstärke und Farbort der Lösungen verändern.

Weiterer Gegenstand der vorliegenden Erfindung sind Formulierungen, insbesondere flüssige Formulierungen, enthaltend mindestens eine Trisazoverbindung der Formel (I) und die Verwendung dieser Formulierungen als Färbezusammensetzung für Färbe- und Druckanwendungen, insbesondere als Aufzeichnungsflüssigkeiten für den Tintenstrahldruck und für Schreibgeräte.

Die erfindungsgemäßen flüssigen Formulierung enthalten im Allgemeinen 0,5 bis 25 Gew.-%, bevorzugt 1,0 bis 15 Gew.-% und besonders bevorzugt 2,0 bis 8,0 Gew.-% an mindestens einer Trisazoverbindung der Formel (I) bezogen auf die gesamte Formulierung.

Die erfindungsgemäßen Formulierung sind vorzugsweise wasserbasiert. Sie enthalten im Allgemeinen40 bis 99 Gew.-%, vorzugsweise 70 bis 95 Gew.-% Wasser und gegebenenfalls einen oder mehrere der folgenden Zusatzstoffe aus der Reihe N-Methyl-2-pyrrolidon, 2-Pyrrolidon, 2-Hexylpyrrolidon, Hydroxyethylpyrrolidon, 2-Propanol, Ethandiol, 1,2-Hexandiol, 1,2-Butandiol, Trimethylolpropan, Diethylenglykol, Diethylenglykolmonobutylether, Triethylenglycolmonobutylether, Dipropylenglykolmonobutylether, Gylcerin, Butyllactat, Harnstoff, Sulfolan, Gylcolether und Biozide, wobei die Gesamtmenge aller Zusatzstoffe 0.01 bis 50 Gew.-%, vorzugsweise 0.1 bis 20 Gew.-%() bezogen auf die gesamte Formulierung beträgt.

Die erfindungsgemäßen flüssigen Formulierungen können auch auf organischen Lösungsmitteln basiert sein. In diesem Fall enthalten die Formulierungen im Allgemeinen 40 bis 99 Gew.-%, vorzugsweise 70bis 95 Gew.-% an mindestens einem Lösungsmittel aus der Reihe N-Methyl-2-pyrrolidon, 2-Pyrrolidon, 2-Hexylpyrrolidon, Hydroxyethylpyrrolidon, 2-Propanol, Ethandiol, 1,2-Hexandiol, 1,2-Butandiol, Trimethylolpropan, Diethylenglykol, Diethylenglykolmonobutylether, Triethylenglycolmonobutylether, Dipropylenglykolmonobutylether, Gylcerin, Butyllactat und gegebenenfalls einen oder mehrere der folgenden Zusatzstoffe aus der Reihe Harnstoff, Sulfolan, Gylcolether, Biozide, wobei die Gesamtmenge aller Zusatzstoffe 0.01 bis 50 Gew.-%, vorzugsweise 0.1 bis 20 Gew.-% bezogen auf die gesamte Formulierung beträgt.

Die erfindungsgemäßen Formulierungen können hergestellt werden indem man mindestens eine erfindungsgemäße Trisazoverbindung der Formel (I) mit Wasser und/oder mindestens einem organischen Lösungsmittel und gegebenenfalls mit einem oder mehreren Zusatzstoffen mischt.

Weiterer Gegenstand der vorliegenden Erfindung ist die Verwendung einer erfindungsgemäßen Formulierung als Färbezusammensetzung, insbesondere Tinte, Aufzeichnungsflüssigkeit oder Textilfärbemittel.

Die erfindungsgemäßen Trisazoverbindungen der Formel (I) können hergestellt werden durch Umsetzung mindestens einer Verbindung der Formel (II) 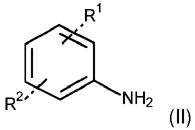 worin
R¹ und R² die für Formel (I) angegebenen Bedeutungen haben,
mit einem Diazotierungsreagenz
und anschließender Umsetzung der Reaktionsmischung mit mindestens einer Verbindung der Formel (III) 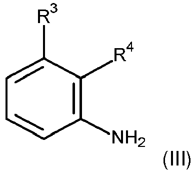 worin
   R³ und R⁴ die für Formel (I) angegebenen Bedeutungen haben,
   zu dem Zwischenprodukt der (Formel IV) 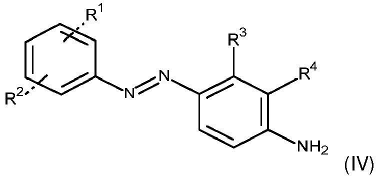 worin
      R¹, R², R³ und R⁴ die für Formel (I) angegebenen Bedeutungen haben, und weiterer Umsetzung des Zwischenprodukts der Formel (IV) mit einem Diazotierungsreagenz und anschließender Umsetzung der so erhaltenen Reaktionsmischung mit
      einer Verbindung der Formel (V) 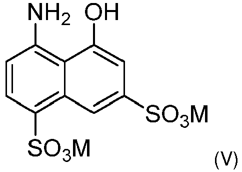
      unter Bildung des Zwischenprodukts der Formel (VI) 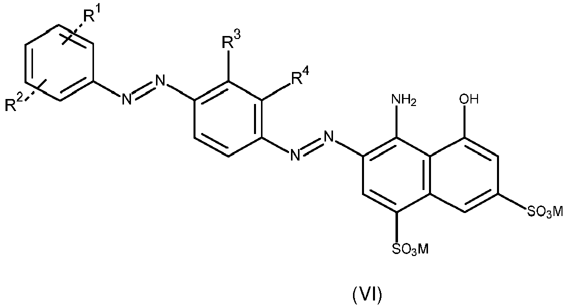 worin
         R¹, R², R³ und R⁴ die für Formel (I) angegebenen Bedeutungen haben,
         nachfolgender Umsetzung des Zwischenprodukts der Formel (VI) mit dem Umsetzungsprodukt das erhältlich ist durch Umsetzung einer Verbindung der Formel (VII) 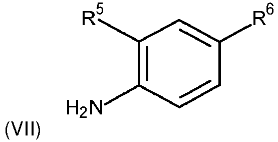
         worin R⁵ und R⁶ die für Formel (I) angegebenen Bedeutungen haben, mit einem Diazotierungsreagenz.

Als Diazotierungsreagenz eignen sich anorganische und organische Nitrite, Nitrosylschwefelsäure vorzugsweise Natriumnitrit oder Methylnitrit.

Die Durchführung der Diazotierungsschritte des erfindungsgemäßen Verfahrens erfolgt in dem Fachmann bekannter Art und Weise. Der pH-Wert für die Diazotierungschritte kann in einem weiten Bereich variiert werden. Üblicherweise kann der pH-Wert im Bereich von 0 bis 12 liegen.

Das Diazotierungsreagenz kann einzeln oder in beliebiger Mischung untereinander eingesetzt werden. Das erfindungsgemäße Herstellverfahren wird üblicherweise in einem Temperaturbereich von -20°C bis +90°C, vorzugsweise von -10°C bis +60°C durchgeführt.

Zweckmäßigerweise werden die einzelnen Schritte des erfindungsgemäßen Verfahrens bei Umgebungsdruck vorgenommen, man kann die Umsetzung jedoch auch im Bereich von 1000 bis 10000 hPa, vorzugsweise 10 bis 5000 hPa durchführen. Unter Umgebungsdruck ist ein Luftdruck im Bereich von etwa 925 hPa bis 1070 hPa zu verstehen.

Die erfindungsgemäßen Trisazoverbindungen werden üblicherweise nicht isoliert, sondern direkt in der entstehenden, wässrigen Lösung weiterverwendet. Eine Aufreinigung der Produktlösung z.B. durch den Einsatz von Ionentauschern oder Druckpermeation ist möglich aber nicht zwingend notwendig. Zur Aufarbeitung und Isolierung der erfindungsgemäßen Trisazofarbstoffe ist es aber möglich diese mit Ethanol zu fällen und auf einer Filternutsche zu waschen.

Die folgenden Beispiele sollen die vorliegende Erfindung erläutern ohne sie jedoch darauf zu beschränken.

### Beispiele:

### Beispiel A (nicht erfindungsgemäß)

Beispiel A ist der nicht erfindungsgemäße Farbstoff C.I. Acid Black 1 entsprechend der Formel 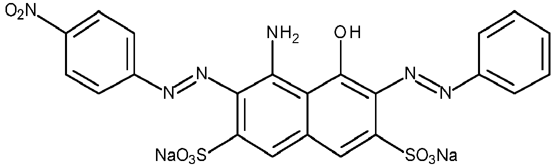 Handelsprodukt der Firma TCI Chemicals.

### Beispiel B (nicht erfindungsgemäß)

Beispiel B ist der nicht erfindungsgemäße Farbstoff C.I. Direct Black 19 entsprechend der Formel 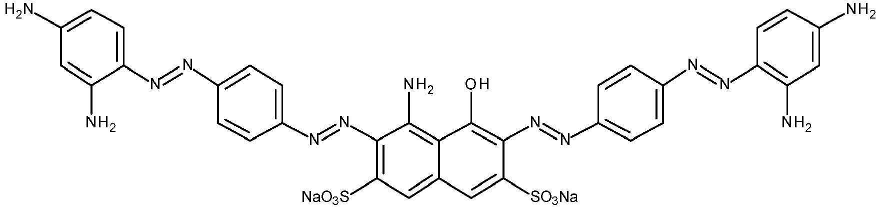 Handelsprodukt der Firma Dystar unter dem Handelsnamen Jettex Direct Black 19.

### Beispiel 1: (nicht erfindungsgemäß)

1,00 mmol 5-Aminoisophtahlsäurewurden in 80 Äquivalenten Wasser suspendiert. Anschließend wurden 2,50 Äquivalente Salzsäure in Form einer 37%igen wässrigen Lösung hinzugefügt und das Reaktionsgemisch auf 2 °C abgekühlt. Eine 40%ige wässrige Natriumnitrit-Lösung wurde in äquimolarer Menge hinzugefügt und die Mischung 1 Stunde bei 3 °C gerührt. Der Nitrit Überschuss wurde durch Zugabe von Amidosulfonsäure entfernt. Anschließend wurde eine äquimolare Menge m-Toluidin in Wasser suspendiert und innerhalb von 60 Minuten zu dem Reaktionsgemisch hinzugefügt. Anschließend wurde der pH-Wert der Reaktionsmischung mit Salzsäure auf pH 1,8 eingestellt und für 30 Minuten gerührt. Der schwarze Niederschlag wurde durch Filtration isoliert und mit salzsaurem Wasser (1%ige wässrige HCl-Lösung) gewaschen.

Der Filterkuchen wurde in 80 Äquivalenten Wasser suspendiert und bei 4 °C für 40 Minuten gerührt.

Anschließend wurden 2,75 Äquivalente HCl zugegeben und anschließend über einen Zeitraum von 60 Minuten eine äquimolare Menge einer 40%igen wässrigen Natriumnitrit-Lösung zudosiert. Der Nitrit Überschuss wurde durch Zugabe von Amidosulfonsäure entfernt. Anschließend wurde eine äquimolare Menge 4-Amino-5-hydroxynaphthalin-1,7-disulfonsäure zugegeben und die Suspension für 2 Stunden gerührt. Die Suspension wurde auf 15 °C erwärmt und der pH-Wert durch Zugabe wässriger HCl Lösung auf 1,1 eingestellt. Anschließend wurde das Reaktionsgemisch für 12 Stunden gerührt und der pH-Wert mit einer wässrigen NaOH-Lösung auf 7,7 gestellt (Suspension 1). Eine äquimolaren Menge 4-[(4-Amino-3-nitrophenyl)amino]-4-oxobutansäurewurde in 70 Äquivalenten Wasser suspendiert und 4 Äquivalente Salzsäure hinzugefügt. Anschließend wurde eine äquimolare Menge einer wässrigen Natriumnitrit-Lösung hinzugefügt. Der Nitrit Überschuss wurde durch Zugabe von Amidosulfonsäure entfernt. Zu dieser Suspension wurde die Suspension 1 bei einer Temperatur von 3 °C zudosiert. Der pH Wert wurde mit wässriger NaOH-Lösung auf 8,8 eingestellt. Anschließend wurde das Reaktionsgemisch auf Raumtemperatur erwärmt. Zur weiteren Aufreinigung wurde über ein SiO₂-Bett (Celite ^{®}) filtriert.

Es wurden 0.42 mmol des Farbstoffs der Formel 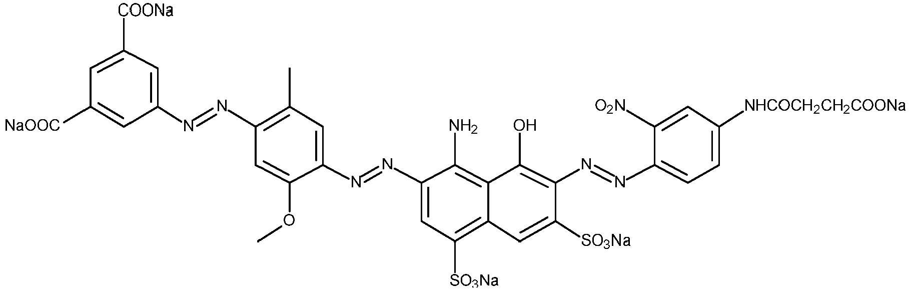 erhalten. Ausbeute:42 %

### Beispiel 2: (nicht erfindungsgemäß)

Die Herstellung des Farbstoffs des Beispiels 2 erfolgte analog zu Beispiel 1, mit dem Unterschied, dass anstelle von 5-Aminoisophtahlsäure eine äquimolare Mengen p-Aminobenzoesäure eingesetzt wurden. Es wurden 0.44 mmol des Farbstoffes der Formel 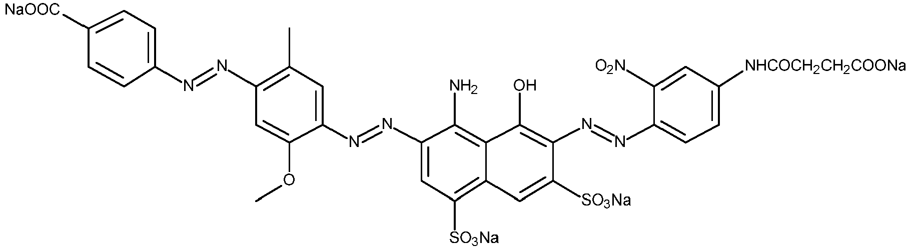 erhalten. Ausbeute: 44 %

### Beispiel 3:

Die Herstellung des Farbstoffs des Beispiels 3 erfolge analog zu Beispiel 1, mit dem Unterschied, dass anstelle von 5-Aminoisophthalsäure eine äquimolare Menge Anilin-2,4-disulfonsäure und anstelle von 4-[(4-Amino-3-nitrophenyl)amino]-4-oxobutansäure eine äquimolare Menge 4-Nitroanilin-2-sulfonsäure eingesetzt wurden. Es wurden 0.49 mmol des Farbstoffes der Formel 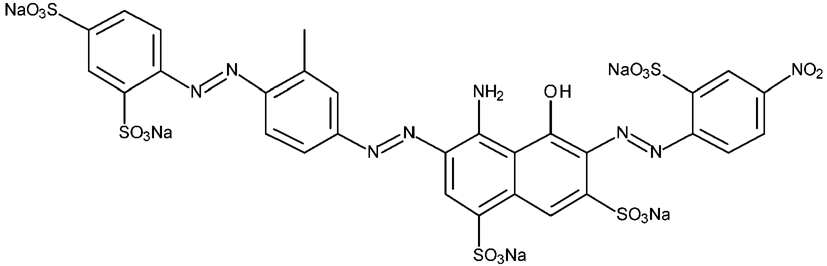 erhalten. Ausbeute 49%

### Beispiel 4:

Die Herstellung des Farbstoffs des Beispiels 4 erfolge analog zu Beispiel 1, mit dem Unterschied, dass anstelle von 5-Aminoisophthalsäure eine äquimolare Menge Anilin-2,4-disulfonsäure und anstelle von 4-[(4-Amino-3-nitrophenyl)amino]-4-oxobutansäure eine äquimolare Menge 2-Nitroanilin-4-sulfonsäure eingesetzt wurden. Es wurden 0.50 mmol des Farbstoffes der Formel 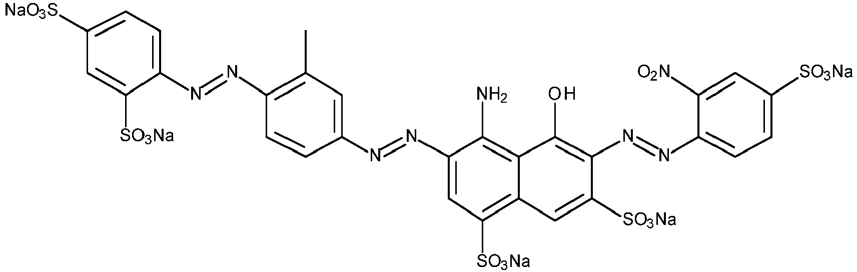 erhalten. Ausbeute 50%

### Beispiel 5:

Die Herstellung des Farbstoffs des Beispiel 5 erfolge analog zu Beispiel 1, mit dem Unterschied, dass anstelle von 5-Aminoisophthalsäure eine äquimolare Menge Anilin-2,4-disulfonsäure, anstelle von 4-[(4-Amino-3-nitrophenyl)amino]-4-oxobutansäure eine äquimolare Menge 2-Nitroanilin-4-sulfonsäure und anstelle von *m*_Toluidin eine äquimolare Menge *o*-Toluidin eingesetzt wurde. Es wurden 0.53 mmol des Farbstoffes der Formel 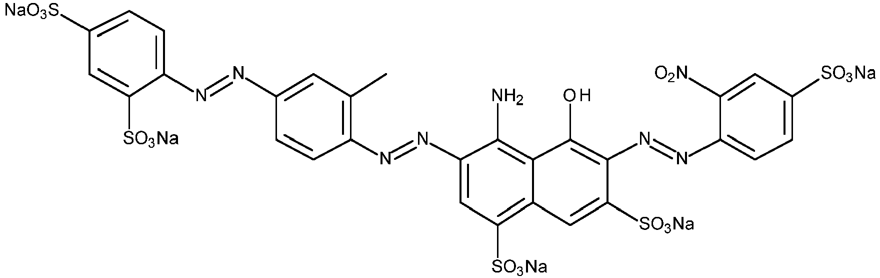 erhalten. Ausbeute 53%

### Beispiel 6:

Die Herstellung des Farbstoffs des Beispiels 6 erfolge analog zu Beispiel 1, mit dem Unterschied, dass anstelle von 5-Aminoisophthalsäure eine äquimolare Menge Anilin-2,4-disulfonsäure, anstelle von 4-[(4-Amino-3-nitrophenyl)amino]-4-oxobutansäure eine äquimolare Menge 4-Nitroanilin-2-sulfonsäure und anstelle von *m*_Toluidin eine äquimolare Menge *o*-Toluidin eingesetzt wurde. Es wurden 0.48 mmol des Farbstoffes der Formel 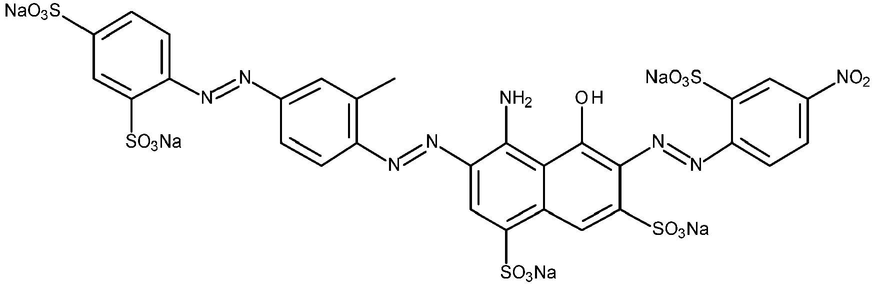 erhalten. Ausbeute 48%

### Beispiel 7:

Die Herstellung des Farbstoffs des Beispiels 7 erfolge analog zu Beispiel 1, mit dem Unterschied, dass anstelle von 5-Aminoisophthalsäure eine äquimolare Menge Anilin-2,5-disulfonsäure und anstelle von 4-[(4-Amino-3-nitrophenyl)amino]-4-oxobutansäure eine äquimolare Menge 4-Nitroanilin-2-sulfonsäure eingesetzt wurde. Es wurden 0.42 mmol des Farbstoffes der Formel 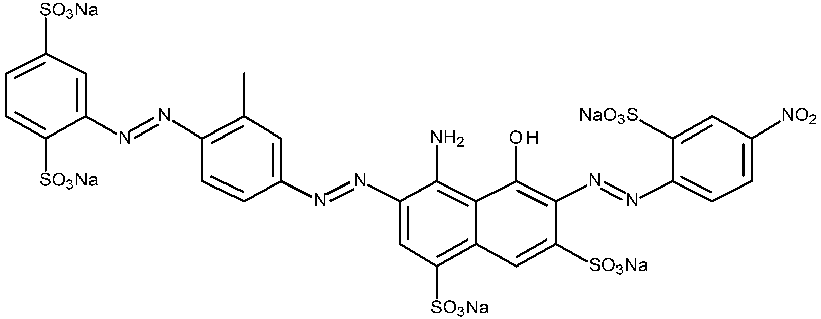 erhalten. Ausbeute 42%

### Beispiel 8:

Die Herstellung des Farbstoffs des Beispiels 8 erfolge analog zu Beispiel 1, mit dem Unterschied, dass anstelle von 5-Aminoisophthalsäure eine äquimolare Menge Anilin-2,5-disulfonsäure und anstelle von 4-[(4-Amino-3-nitrophenyl)amino]-4-oxobutansäure eine äquimolare Menge 2-Nitroanilin-4-sulfonsäure eingesetzt wurde. Es wurden 0.49 mmol des Farbstoffes der Formel 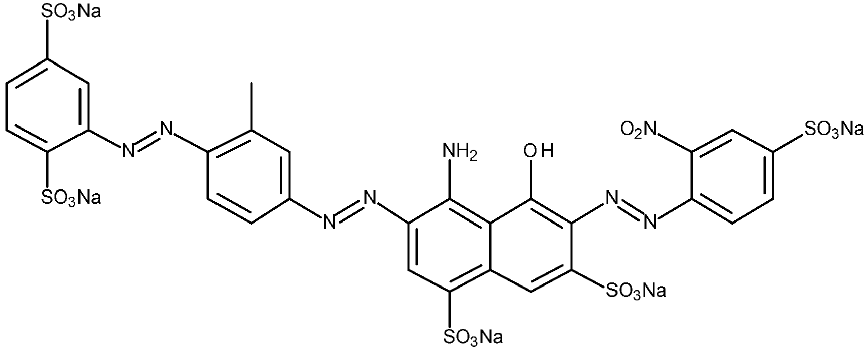 erhalten. Ausbeute 49%

### Beispiel 9:

Die Herstellung des Farbstoffs des Beispiels 9 erfolge analog zu Beispiel 1, mit dem Unterschied, dass anstelle von 5-Aminoisophthalsäure eine äquimolare Menge Anilin-2,5-disulfonsäure, anstelle von 4-[(4-Amino-3-nitrophenyl)amino]-4-oxobutansäure eine äquimolare Menge 2-Nitroanilin-4-sulfonsäure und anstelle von *m*_Toluidin eine äquimolare Menge *o*-Toluidin eingesetzt wurde. Es wurden 0.53 mmol des Farbstoffes der Formel 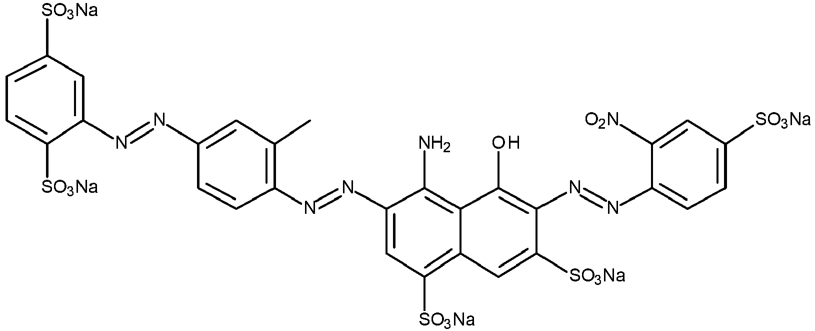 erhalten. Ausbeute 53%

### Beispiel 10:

Die Herstellung des Farbstoffs des Beispiels 10 erfolge analog zu Beispiel 1, mit dem Unterschied, dass anstelle von 5-Aminoisophthalsäure eine äquimolare Menge Anilin-2,5-disulfonsäure, anstelle von 4-[(4-Amino-3-nitrophenyl)amino]-4-oxobutansäure eine äquimolare Menge 4-Nitroanilin-2-sulfonsäure und anstelle von *m*_Toluidin eine äquimolare Menge *o*-Toluidin eingesetzt wurde. Es wurden 0.50 mmol des Farbstoffes der Formel 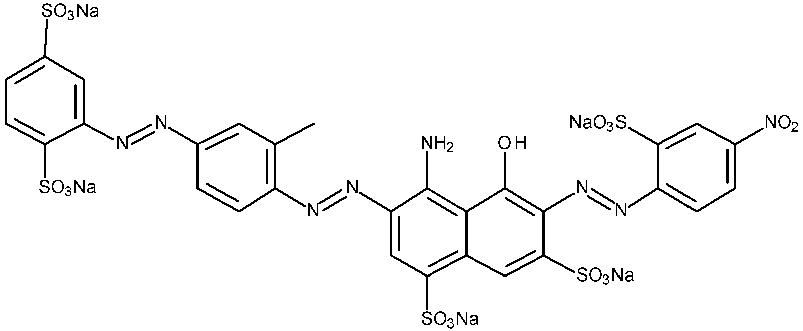 erhalten. Ausbeute 50%

### Beispiel 11:

Die Herstellung des Farbstoffs des Beispiels 11 erfolge analog zu Beispiel 1, mit dem Unterschied, dass anstelle von 5-Aminoisophthalsäure eine äquimolare Menge Anilin-2,5-disulfonsäure, anstelle von 4-[(4-Amino-3-nitrophenyl)amino]-4-oxobutansäure eine äquimolare Menge 4-Nitroanilin-2-sulfonsäure und anstelle von *m*_Toluidin eine äquimolare Menge *o*-Toluidin eingesetzt wurde. Im Anschluss an die Synthese werden 10.0 Äquivalente Ammoniumchlorid zugesetzt und das Reaktionsgemisch für 2h bei Raumtemperatur gerührt. Zur weiteren Aufreinigung wurde über ein SiO₂-Bett (Celite ^{®}) filtriert. Es wurden 0.49 mmol des Farbstoffes der Formel 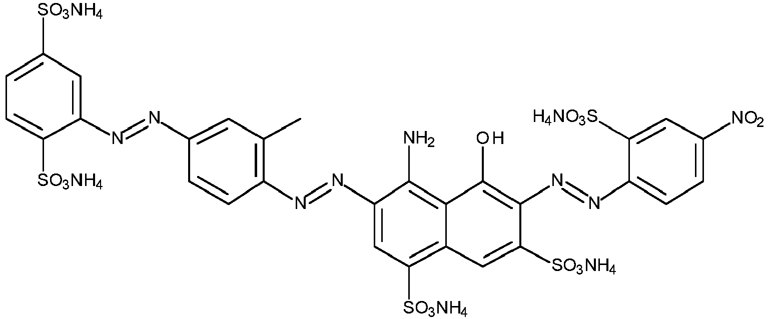 erhalten. Ausbeute 49%

### Beispiel 12:

Die Herstellung des Farbstoffs des Beispiels 11 erfolge analog zu Beispiel 1, mit dem Unterschied, dass anstelle von 5-Aminoisophthalsäure eine äquimolare Menge Anilin-2,5-disulfonsäure, anstelle von 4-[(4-Amino-3-nitrophenyl)amino]-4-oxobutansäure eine äquimolare Menge 4-Nitroanilin-2-sulfonsäure und anstelle von *m*_Toluidin eine äquimolare Menge *o*-Toluidin eingesetzt wurde. Im Anschluss an die Synthese werden 10.0 Äquivalente Triethylamin zugesetzt und das Reaktionsgemisch für 2h bei Raumtemperatur gerührt. Zur weiteren Aufreinigung wurde über ein SiO₂-Bett (Celite ^{®}) filtriert. Es wurden 0.47 mmol des Farbstoffes der Formel 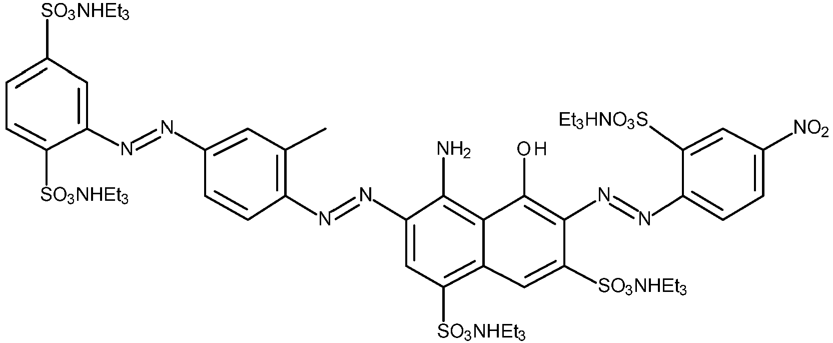 erhalten. Ausbeute 47%

### Beispiel 13:

Die Herstellung des Farbstoffs des Beispiels 13 erfolge analog zu Beispiel 1, mit dem Unterschied, dass anstelle von 5-Aminoisophthalsäure eine äquimolare Menge 4-Aminophthalsäure und anstelle von 4-[(4-Amino-3-nitrophenyl)amino]-4-oxobutansäure eine äquimolare Menge 2-Amino-4-nitrobenzoesäure eingesetzt wurden. Es wurden 0.50 mmol des Farbstoffes der Formel 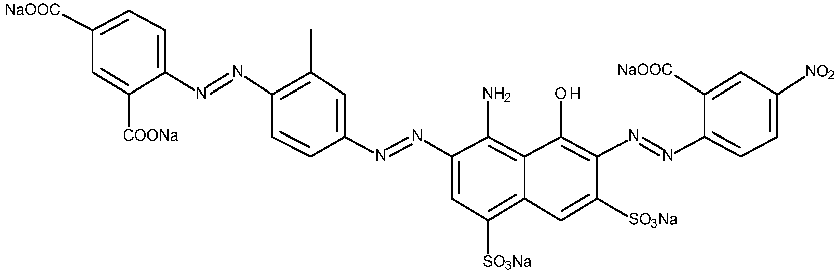 erhalten. Ausbeute 50%

### Beispiel 14:

Die Herstellung des Farbstoffs des Beispiels 13 erfolge analog zu Beispiel 1, mit dem Unterschied, dass anstelle von 5-Aminoisophthalsäure eine äquimolare Menge 4-Aminophthalsäure und anstelle von 4-[(4-Amino-3-nitrophenyl)amino]-4-oxobutansäure eine äquimolare Menge 4-Nitroanilin-2-sulfonsäure eingesetzt wurden. Es wurden 0.52 mmol des Farbstoffes der Formel 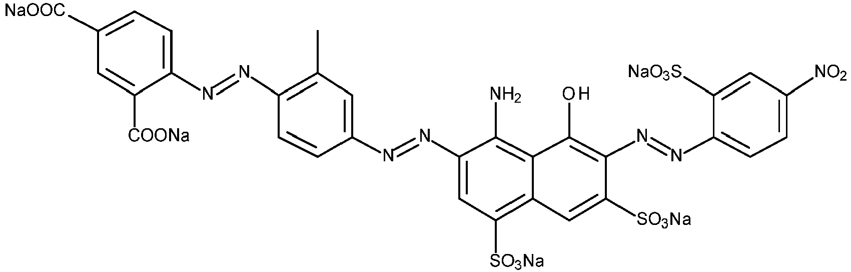 erhalten. Ausbeute 52%

### Bestimmung der Lagerstabilität einer wässrigen Farbstofflösung:

Der angegebene E1/1 Wert ist ein fiktiver Extinktionswert, den man erhalten würde, wenn man eine 1 gewichtsprozentige Lösung der jeweiligen Verbindung (gelöst in Wasser) in einer Küvette der Schichtdicke von 1 cm vermessen würde.

Die Veränderung des E1/1 Wertes, sowie die Änderung des λₘₐₓ Wertes dienen als Maß für die Lagerstabilität einer wässrigen Farbstofflösung. Zur Bestimmung der Lagerstabilität der erfindungsgemäßen und nicht erfindungsgemäßen Farbstoffe der Beispiele A und B sowie 1 bis 14 wurden jeweils wässrige Farbstofflösungen mit einer Konzentration von 19 Gew.-% hergestellt und diese Farbstofflösungen für 28 Tage bei 45 °C in einem geschlossenen Glasgefäß gelagert. Der E1/1 Wert wurde jeweils nach 0 Tagen und nach 28 Tagen entsprechend der oben beschriebenen Vorschrift gemessen. Die Veränderung des E1/1 Werts über diesen Zeitraum ergibt den Wert ΔE1/1 = E1/1 (28 Tage) - E1/1 (0 Tage). Die Veränderung des λₘₐₓ Werts über diesen Zeitraum ergibt den Wert Δλₘₐₓ = λₘₐₓ (28 Tage) - λₘₐₓ (0 Tage).

Die Veränderung des E1/1 Wertes ist dabei ein Maß für den Farbstärkeverlust über den Lagerungszeitraum, die Veränderung des λₘₐₓ Wertes ist ein Maß für die Verschiebung des Farbortes über den Lagerungszeitraum.

Die Ergebnisse sind in Tabelle 1 wiedergegeben.

**Tabelle 1**

| **Beispiel** | **erfindungsgemäß** | **λₘₐₓ** | **Δ E1/1 bei λₘₐₓ** | **Δ λₘₐₓ** |
|---|---|---|---|---|
| | | **[nm]** | | **[nm]** |
| A | nein | 619 | -14 | 5 |
| B | nein | 618 | -11 | -6 |
| 1 | nein | 621 | -7 | -8 |
| 2 | nein | 622 | -9 | -5 |
| 3 | ja | 596 | <-1 | 0 |
| 4 | ja | 615 | -3 | -1 |
| 5 | ja | 616 | <-1 | 1 |
| 6 | ja | 595 | -3 | 0 |
| 7 | ja | 596 | <-1 | 1 |
| 8 | ja | 613 | -3 | -1 |
| 9 | ja | 613 | -2 | 1 |
| 10 | ja | 598 | -3 | 1 |
| 11 | ja | 598 | -2 | -1 |
| 12 | ja | 600 | -2 | 0 |
| 13 | ja | 625 | -3 | 1 |
| 14 | ja | 619 | <-1 | -1 |

| | | | | |
|---|---|---|---|---|
| Fazit: Wie aus Tabelle 1 ersichtlich ist zeigen die wässrigen Lösungen der erfindungsgemäßen Trisazoverbindungen der Beispiele 3 bis 14 auch nach längerer Lagerung über einen Zeitraum von 28 Tagen bei einer erhöhten Temperatur von 45 °C eine deutlich geringere Veränderung der Extinktionswerte sowie der λₘₐₓ Werte gegenüber den nicht erfindungsgemäßen Farbstoffen der Beispiele A und B sowie 1 und 2 des Standes der Technik. Das bedeutet, bei den wässrigen Lösungen der erfindungsgemäßen Trisazoverbindungen bleiben Farbstärke und Farbort über einen Zeitraum von 28 Tagen nahezu unverändert, wo hingegen bei den nicht erfindungsgemäßen Farbstoffen deutliche Abweichungen feststellbar sind. | | | | |

## Patentansprüche

1. Trisazoverbindungen der Formel (I) 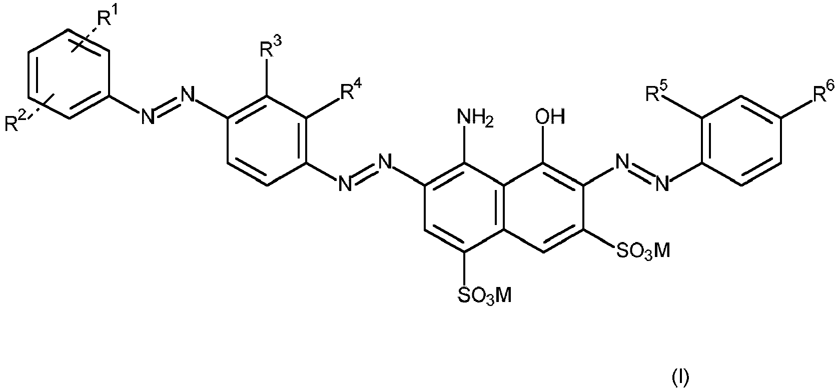 worin
R¹ für SO₃M oder COOM steht, und
R² für SO₃M oder COOM steht,
R³ und R⁴ unabhängig voneinander für Wasserstoff oder CH₃ stehen,
R⁵ und R⁶ unabhängig voneinander für SO₃M, COOM oder NO₂ stehen,
und
M für Wasserstoff, ein einwertiges Metallkation, für Ammonium oder für Alkylammonium, welches ein- oder mehrfach, gleich oder verschieden durch C₁-C₄-Alkyl substituiert ist, steht.

2. Trisazoverbindungen gemäß Anspruch 1, **dadurch gekennzeichnet, dass**
R¹ für SO₃M oder COOM steht,
R² für SO₃M oder COOM steht,
R³ und R⁴ unabhängig voneinander für Wasserstoff oder CH₃ stehen, und
R⁵ und R⁶ unabhängig voneinander für SO₃M, COOM oder NO₂ stehen,
mit der Maßgabe, dass einer der Reste R⁵ oder R⁶ für NO₂ steht,
und
M für Wasserstoff, Natrium, Kalium, Lithium, Ammonium, oder Alkylammonium, welches ein- oder mehrfach, gleich oder verschieden durch C₁-C₂-Alkyl substituiert ist, steht.

3. Trisazoverbindungen gemäß Anspruch 1 oder 2, **dadurch gekennzeichnet, dass**
R1 für SO3M oder COOM steht,
R² für SO₃M oder COOM steht,
R³ und R⁴ unabhängig voneinander für Wasserstoff oder CH₃ stehen,
und
R⁵ und R⁶ unabhängig voneinander für SO₃M, COOM oder NO₂ stehen,
mit der Maßgabe, dass einer der Reste R⁵ oder R⁶ für NO₂ steht, und
M für Wasserstoff, Natrium, Kalium, Lithium, Ammonium, Trimethylammonium oder Triethylammonium steht.

4. Trisazoverbindungen gemäß einem der Ansprüche 1 bis 3, **dadurch gekennzeichnet, dass**
R¹ für SO₃M oder COOM steht,
R² für SO₃M oder COOM steht,
R³ und R⁴ für Wasserstoff oder CH₃ stehen,
mit der Maßgabe, dass R³ und R⁴ nicht beide gleichzeitig für Wasserstoff und nicht beide gleichzeitig für CH₃ stehen,
R⁵ und R⁶ für SO₃M, COOM oder NO₂ stehen,
mit der Maßgabe, dass einer der Reste R⁵ oder R⁶ für NOz steht,
und
M für Wasserstoff, Natrium, Kalium, Lithium, Ammonium, Trimethylammonium oder Triethylammonium steht.

5. Trisazoverbindungen gemäß einem der Ansprüche 1 bis 4, **dadurch gekennzeichnet, dass** sie den Formeln (la) oder (Ib) 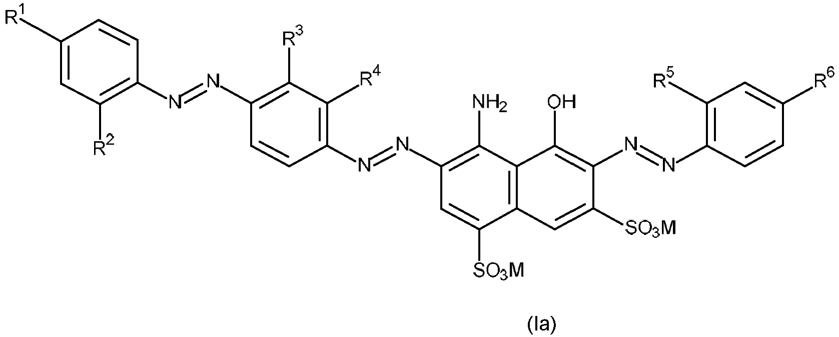 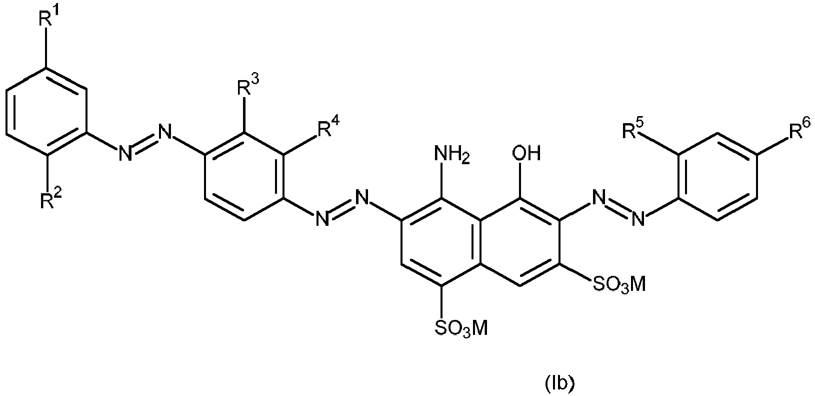 entsprechen,
worin
R¹ für SO₃M oder COOM steht,
R² für SO₃M oder COOM steht,
R³ und R⁴ für Wasserstoff oder CH₃ stehen,
mit der Maßgabe, dass R³ und R⁴ nicht beide gleichzeitig für Wasserstoff und nicht beide gleichzeitig für CH₃ stehen,
R⁵ und R⁶ für SO₃M, COOM oder NOz stehen,
mit der Maßgabe, dass einer der Reste R⁵ oder R⁶ für NOz steht,
und
M für Wasserstoff, Natrium, Kalium, Lithium, Ammonium, Trimethylammonium oder Triethylammonium steht.

6. Trisazoverbindungen gemäß einem der Ansprüche 1 bis 5, **dadurch gekennzeichnet, dass**
R¹ für SO₃M steht,
R² für SO₃M steht,
R³ und R⁴ für Wasserstoff oder CH₃ stehen,
mit der Maßgabe, dass R³ und R⁴ nicht beide gleichzeitig für Wasserstoff stehen und nicht beide gleichzeitig für CH₃ stehen,
R⁵ und R⁶ für SO₃M, COOM oder NOz stehen,
mit der Maßgabe, dass einer der Reste R⁵ oder R⁶ für NOz steht,
und
M für Wasserstoff, Natrium, Kalium, Lithium, Ammonium oder Triethylammonium steht.

7. Trisazoverbindungen gemäß einem der Ansprüche 1 bis 6, **dadurch gekennzeichnet, dass**
R¹ für COOM steht,
R² für COOM steht,
R³ und R⁴ für Wasserstoff oder CH₃ stehen,
mit der Maßgabe, dass R³ und R⁴ nicht beide gleichzeitig für Wasserstoff stehen und nicht beide gleichzeitig für CH₃ stehen,
R⁵ und R⁶ für SO₃M, COOM oder NOz stehen,
mit der Maßgabe, dass einer der Reste R⁵ oder R⁶ für NOz steht,
und
M für Wasserstoff, Natrium, Kalium, Lithium, Ammonium oder Triethylammonium steht.

8. Trisazoverbindungen gemäß einem der Ansprüche 1 bis 7, **dadurch gekennzeichnet, dass** sie der Formel 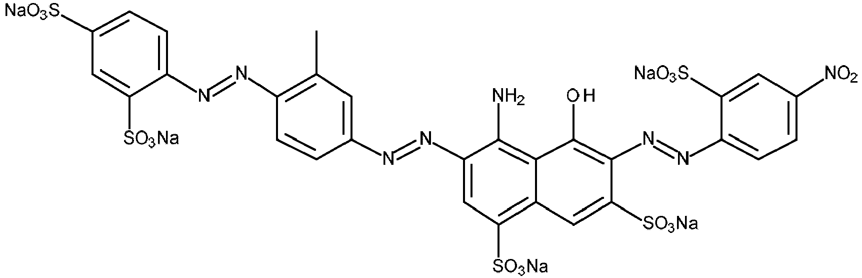 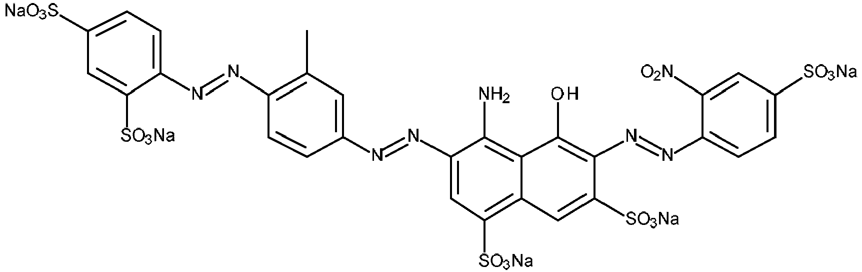 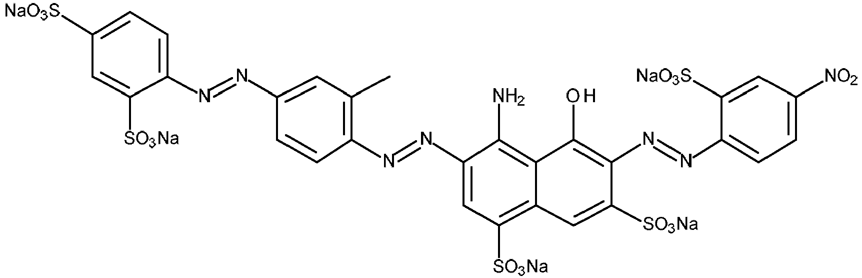 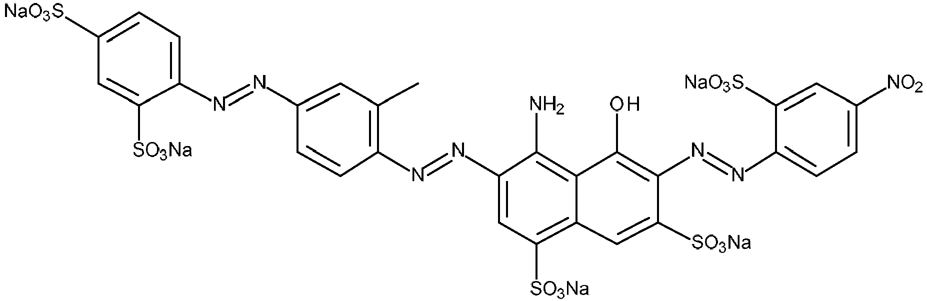 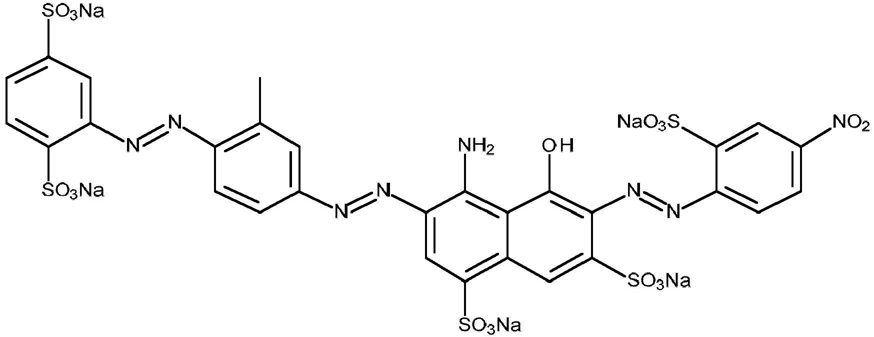 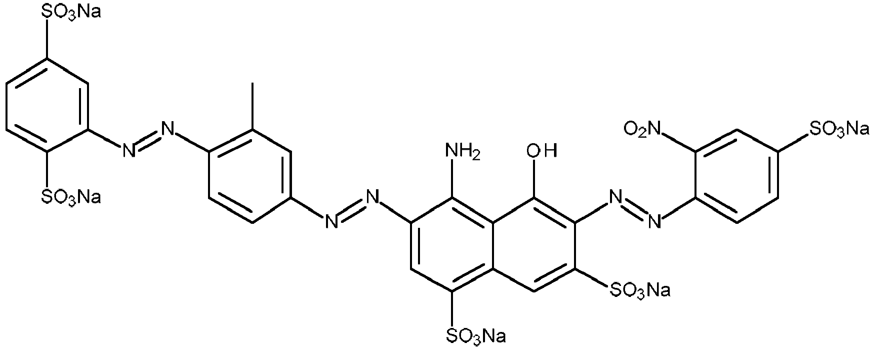 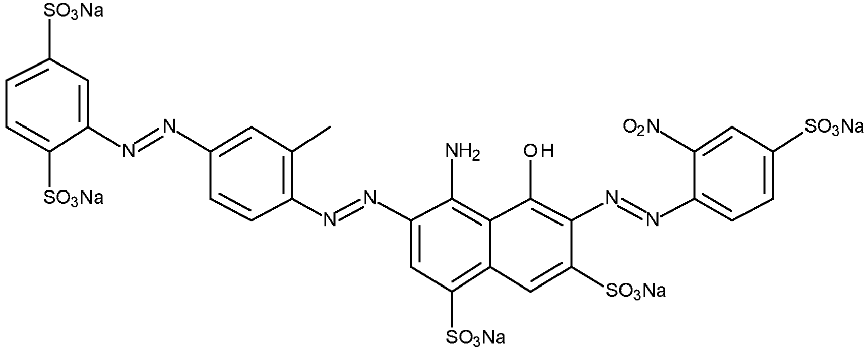 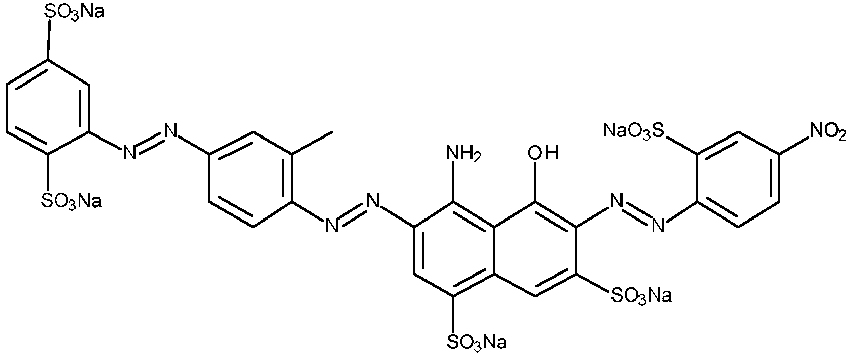 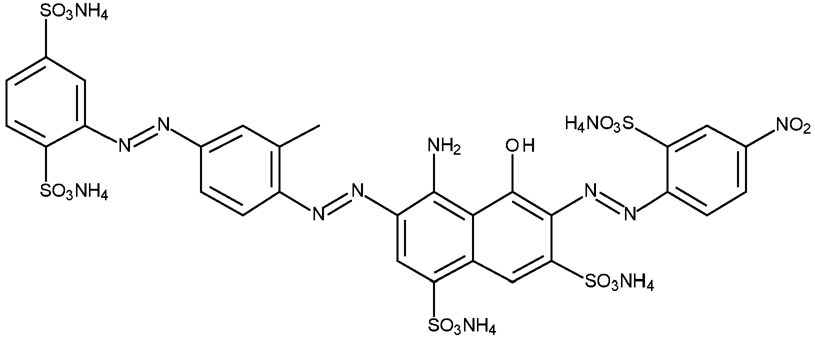 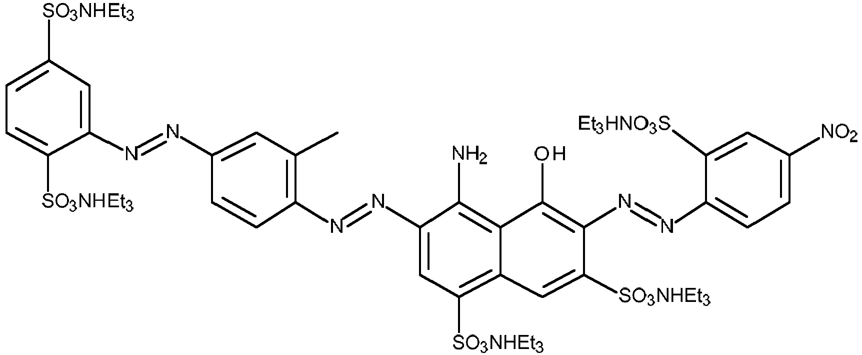 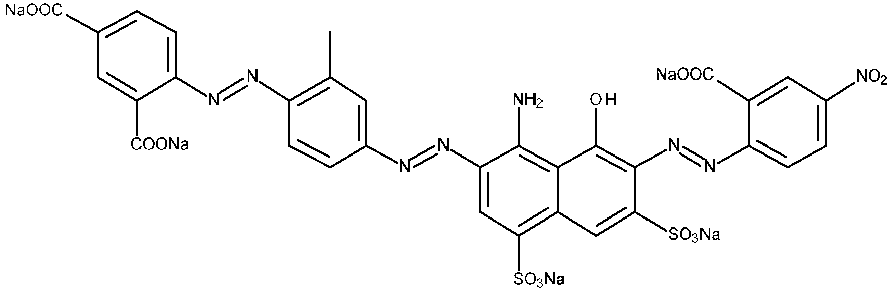 oder 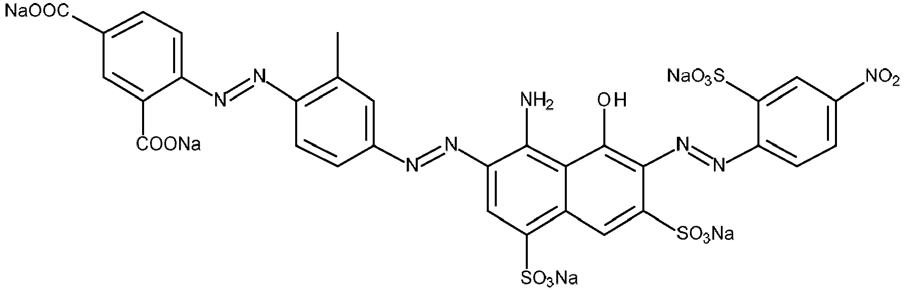 entsprechen.

9. Verfahren zum Färben und Bedrucken von cellulosehaltien Materialien, tierischen Häuten, tierischen Haaren, Eierschalen, porösen Materialien und Metallen, **dadurch gekennzeichnet, dass** als Farbstoff mindestens eine Triazoverbindung gemäß einem der Ansprüche 1 bis 8 verwendet wird.

10. Formulierung enthaltend mindestens eine Trisazoverbindung gemäß einem der Ansprüche 1 bis 8.

11. Formulierung gemäß Anspruch 10, **dadurch gekennzeichnet, dass** es sich um eine flüssige Formulierung handelt.

12. Formulierung gemäß Anspruch 10 oder 11, **dadurch gekennzeichnet, dass** sie 0,5 bis 25 Gew.-%, bevorzugt 1,0 bis 15 Gew.-% und besonders bevorzugt 2,0 bis 8,0 Gew.-% an mindestens einer Trisazoverbindung gemäß einem der Ansprüche 1 bis 8, jeweils bezogen auf die gesamte Formulierung, enthält.

13. Formulierung gemäß einem der Ansprüche 10 bis 12, **dadurch gekennzeichnet, dass** sie 40 bis 99 Gew.-%, vorzugsweise 70 bis 95 Gew.-% Wasser und gegebenenfalls einen oder mehrere Zusatzstoffe aus der Reihe N-Methyl-2-pyrrolidon, 2-Pyrrolidon, 2-Hexylpyrrolidon, Hydroxyethylpyrrolidon, 2-Propanol, Ethandiol, 1,2-Hexandiol, 1,2-Butandiol, Trimethylolpropan, Diethylenglykol, Diethylenglykolmonobutylether, Triethylenglycolmonobutylether, Dipropylenglykolmonobutylether, Gylcerin, Butyllactat, Harnstoff, Sulfolan, Gylcolether und Biozide in einer Gesamtmenge von 0.01 bis 50 Gew.-%, vorzugsweise von 0.1 bis 20 Gew.-% bezogen auf die gesamte Formulierung enthält.

14. Formulierung gemäß einem der Ansprüche 10 bis 13, **dadurch gekennzeichnet, dass** sie 40 bis 99 Gew.-%, vorzugsweise 70 bis 95 Gew.-% an mindestens einem Lösungsmittel aus der Reihe N-Methyl-2-pyrrolidon, 2-Pyrrolidon, 2-Hexylpyrrolidon, Hydroxyethylpyrrolidon, 2-Propanol, Ethandiol, 1,2-Hexandiol, 1,2-Butandiol, Trimethylolpropan, Diethylenglykol, Diethylenglykolmonobutylether, Triethylenglycolmonobutylether, Dipropylenglykolmonobutylether, Gylcerin, Butyllactat und gegebenenfalls einen oder mehrere Zusatzstoffe aus der Reihe Harnstoff, Sulfolan, Gylcolether, Biozide in einer Gesamtmenge von 0.01 bis 50 Gew.-%, vorzugsweise von 0.1 bis 20 Gew.-% bezogen auf die gesamte Formulierung enthält.

15. Verwendung einer Formulierung gemäß einem der Ansprüche 10 bis 14 als Aufzeichnungsflüssigkeit für den Tintenstrahldruck, als Färbemittel in Schreibvorrichtungen oder bei der Herstellung von Farbfiltern für optische und optoelektonische Anwendungen.

16. Verfahren zur Herstellung von Trisazoverbindungen gemäß einem der Ansprüche 1 bis 8, **dadurch gekennzeichnet, dass** mindestens eine Verbindung der Formel (II) 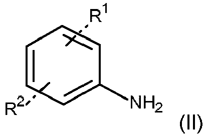 worin
R¹ und R² die in Anspruch 1 angegebenen Bedeutungen haben,
mit einem Diazotierungsreagenz umgesetzt wird und anschließend die erhaltene Reaktionsmischung mit mindestens einer Verbindung der Formel (III) 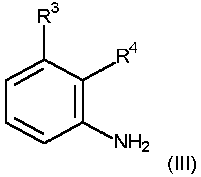 worin
R³ und R⁴ die in Anspruch 1 angegebenen Bedeutungen haben,
zu dem Zwischenprodukt der (Formel IV) 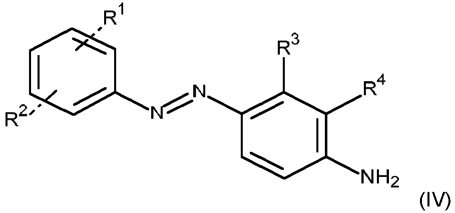 worin
R¹, R², R³ und R⁴ die in Anspruch 1 angegebenen Bedeutungen haben,
umgesetzt wird und anschließend das Zwischenprodukt der Formel (IV) mit einem Diazotierungsreagenz umgesetzt wird und dann die so erhaltene Reaktionsmischung mit einer Verbindung der Formel (V) 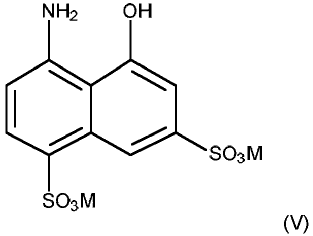
zu dem Zwischenprodukt der Formel (VI) 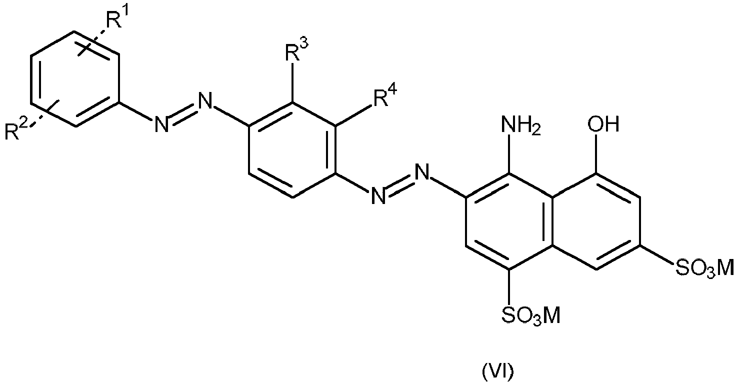 worin
R¹, R², R³ und R⁴ die in Anspruch 1 angegebenen Bedeutungen haben,
umgesetzt wird und nachfolgend das Zwischenprodukt der Formel (VI) weiter umgesetzt wird mit dem Umsetzungsprodukt das erhältlich ist durch Umsetzung einer Verbindung der Formel (VII) 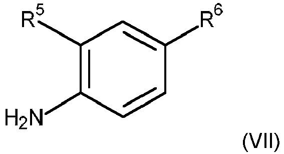 worin
R⁵ und R⁶ die in Anspruch 1 angegebenen Bedeutungen haben,
mit einem Diazotierungsreagenz und anschließend die gebildete Trisazoverbindung der Formel (I) durch Filtration der Reaktionsmischung isoliert wird.

## Claims

1. Trisazo compounds of formula (I) 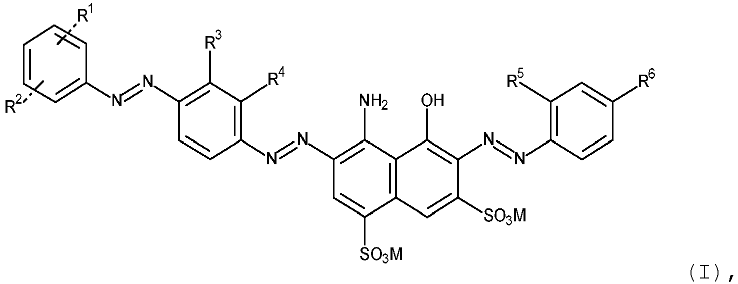 in which
R¹ is SO₃M or COOM, and
R² is SO₃M or COOM,
R³ and R⁴ independently of one another are hydrogen or CH₃,
R⁵ and R⁶ independently of one another are SO₃M, COOM or NO₂,
and
M is hydrogen, a monovalent metal cation, is ammonium or is alkylammonium which is mono- or polysubstituted identically or differently by C₁-C₄-alkyl.

2. Trisazo compounds according to Claim 1, **characterized in that**
R¹ is SO₃M or COOM,
R² is SO₃M or COOM,
R³ and R⁴ independently of one another are hydrogen or CH₃,
and
R⁵ and R⁶ independently of one another are SO₃M, COOM or NO₂,
with the proviso that one of the radicals R⁵ or R⁶ is NO₂,
and
M is hydrogen, sodium, potassium, lithium, ammonium or alkylammonium which is mono- or polysubstituted identically or differently by C₁-C₂-alkyl.

3. Trisazo compounds according to Claim 1 or 2, **characterized in that**
R1 is SO3M or COOM,
R² is SO₃M or COOM,
R³ and R⁴ independently of one another are hydrogen or CH₃,
and
R⁵ and R⁶ independently of one another are SO₃M, COOM or NO₂,
with the proviso that one of the radicals R⁵ or R⁶ is NO₂,
and
M is hydrogen, sodium, potassium, lithium, ammonium, trimethylammonium or triethylammonium.

4. Trisazo compounds according to any of Claims 1 to 3, **characterized in that**
R¹ is SO₃M or COOM,
R² is SO₃M or COOM,
R³ and R⁴ are hydrogen or CH₃,
with the proviso that R³ and R⁴ are not both simultaneously hydrogen and are not both simultaneously CH₃,
R⁵ and R⁶ are SO₃M, COOM or NO₂,
with the proviso that one of the radicals R⁵ or R⁶ is NO₂,
and
M is hydrogen, sodium, potassium, lithium, ammonium, trimethylammonium or triethylammonium.

5. Trisazo compounds according to any of Claims 1 to 4, **characterized in that**
they correspond to formulae (Ia) or (Ib) 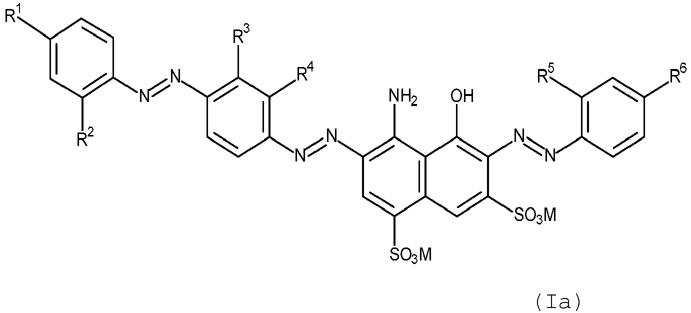 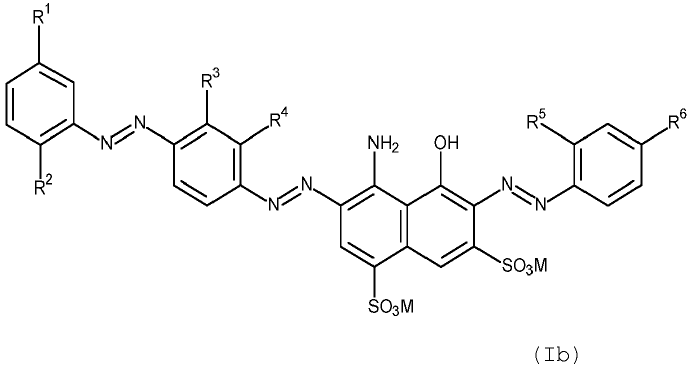 in which
R¹ is SO₃M or COOM,
R² is SO₃M or COOM,
R³ and R⁴ are hydrogen or CH₃,
with the proviso that R³ and R⁴ are not both simultaneously hydrogen and are not both simultaneously CH₃,
R⁵ and R⁶ are SO₃M, COOM or NO₂,
with the proviso that one of the radicals R⁵ or R⁶ is NO₂,
and
M is hydrogen, sodium, potassium, lithium, ammonium, trimethylammonium or triethylammonium.

6. Trisazo compounds according to any of Claims 1 to 5, **characterized in that**
R¹ is SO₃M,
R² is SO₃M,
R³ and R⁴ are hydrogen or CH₃,
with the proviso that R³ and R⁴ are not both simultaneously hydrogen and are not both simultaneously CH₃,
R⁵ and R⁶ are SO₃M, COOM or NO₂,
with the proviso that one of the radicals R⁵ or R⁶ is NO₂,
and
M is hydrogen, sodium, potassium, lithium, ammonium or triethylammonium.

7. Trisazo compounds according to any of Claims 1 to 6, **characterized in that**
R¹ is COOM,
R² is COOM,
R³ and R⁴ are hydrogen or CH₃,
with the proviso that R³ and R⁴ are not both simultaneously hydrogen and are not both simultaneously CH₃,
R⁵ and R⁶ are SO₃M, COOM or NO₂,
with the proviso that one of the radicals R⁵ or R⁶ is NO₂,
and
M is hydrogen, sodium, potassium, lithium, ammonium or triethylammonium.

8. Trisazo compounds according to any of Claims 1 to 7, **characterized in that**
they correspond to the formula 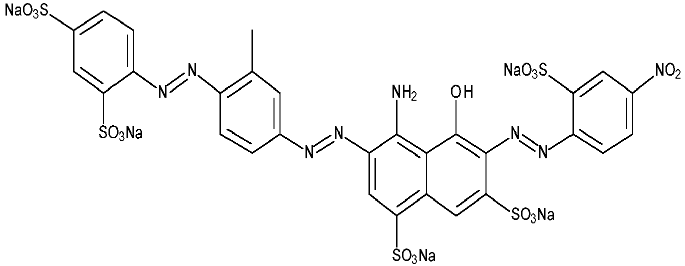 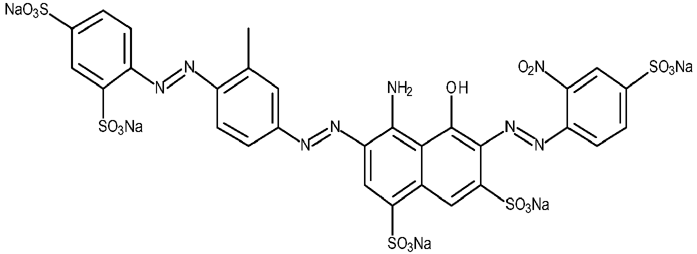 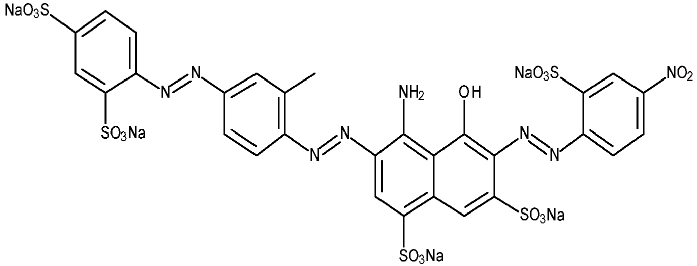 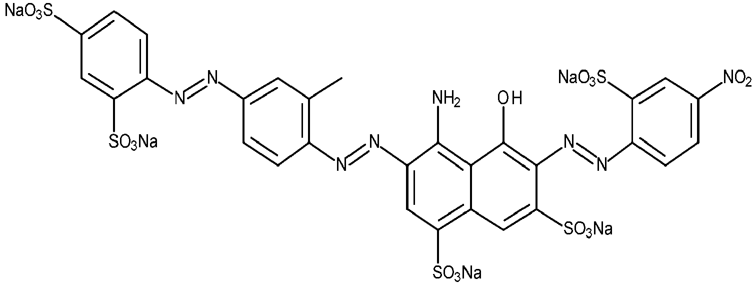 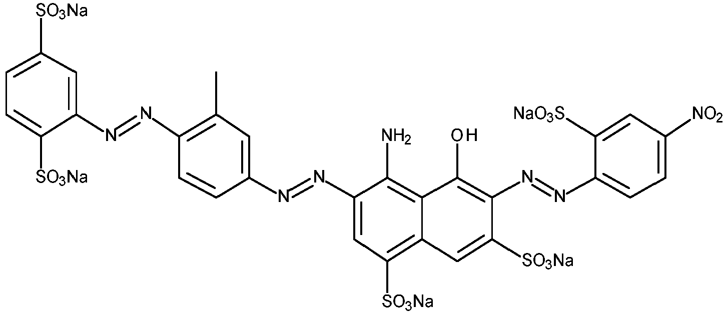 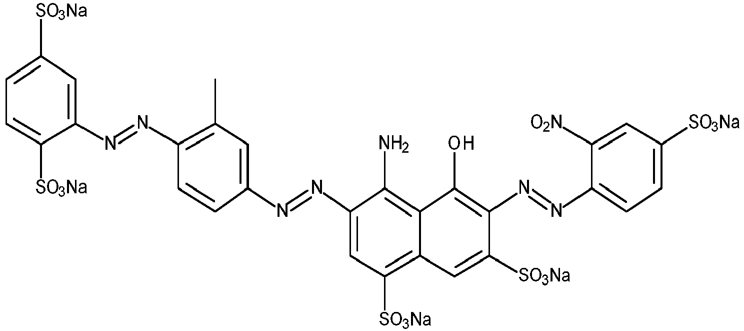 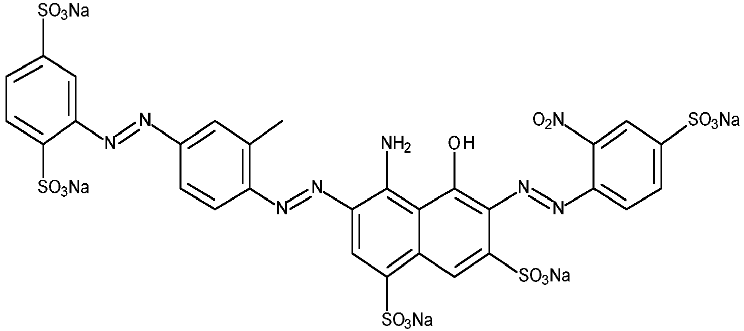 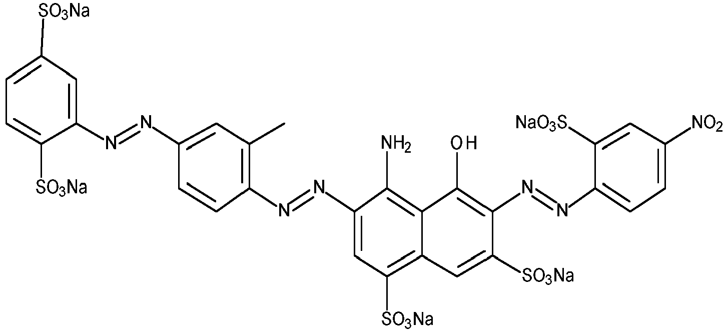 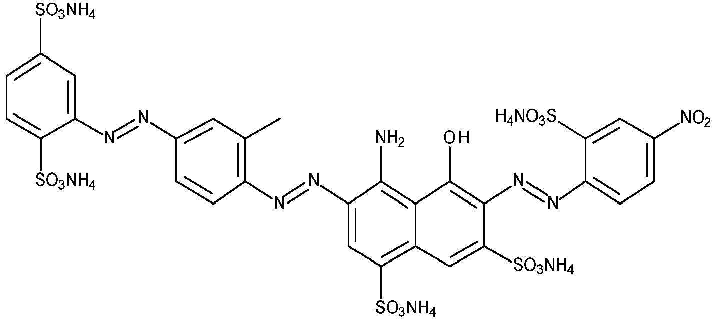 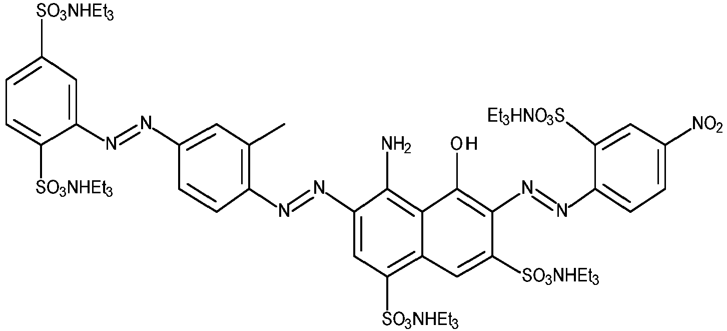 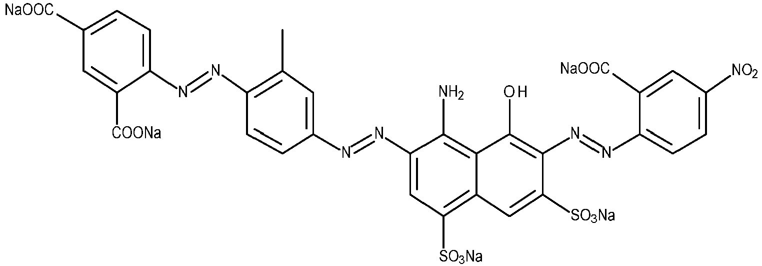 or

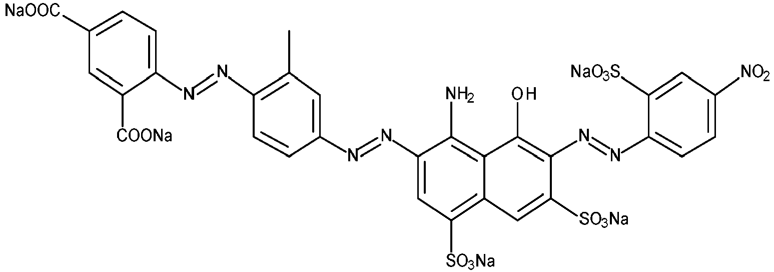

9. Process for the dyeing and printing of cellulose-containing materials, animal hides, animal hair, eggshells, porous materials and metals, **characterized in that** the dye used is at least one triazo compound according to any of Claims 1 to 8.

10. Formulation containing at least one trisazo compound according to any of Claims 1 to 8.

11. Formulation according to Claim 10, **characterized in that** it is a liquid formulation.

12. Formulation according to Claim 10 or 11, **characterized in that** it contains 0.5% to 25% by weight, preferably 1.0% to 15% by weight and particularly preferably 2.0% to 8.0% by weight of at least one trisazo compound according to any of Claims 1 to 8, in each case based on the overall formulation.

13. Formulation according to any of Claims 10 to 12, **characterized in that** it contains 40% to 99% by weight, preferably 70% to 95% by weight of water and optionally one or more additives from the group of N-methyl-2-pyrrolidone, 2-pyrrolidone, 2-hexylpyrrolidone, hydroxyethylpyrrolidone, 2-propanol, ethanediol, hexane-1,2-diol, butane-1,2-diol, trimethylolpropane, diethylene glycol, diethylene glycol monobutyl ether, triethylene glycol monobutyl ether, dipropylene glycol monobutyl ether, glycerol, butyl lactate, urea, sulfolane, glycol ethers and biocides in a total amount of from 0.01% to 50% by weight, preferably of from 0.1% to 20% by weight, based on the overall formulation.

14. Formulation according to any of Claims 10 to 13, **characterized in that** it contains 40% to 99% by weight, preferably 70% to 95% by weight of at least one solvent from the group of N-methyl-2-pyrrolidone, 2-pyrrolidone, 2-hexylpyrrolidone, hydroxyethylpyrrolidone, 2-propanol, ethanediol, hexane-1,2-diol, butane-1,2-diol, trimethylolpropane, diethylene glycol, diethylene glycol monobutyl ether, triethylene glycol monobutyl ether, dipropylene glycol monobutyl ether, glycerol, butyl lactate and optionally one or more additives from the group of urea, sulfolane, glycol ethers, biocides in a total amount of from 0.01% to 50% by weight, preferably of from 0.1% to 20% by weight, based on the overall formulation.

15. Use of a formulation according to any of Claims 10 to 14 as a recording fluid for inkjet printing, as a colourant in writing devices or in the production of colour filters for optical and optoelectronic applications.

16. Process for preparing trisazo compounds according to any of Claims 1 to 8, **characterized in that** at least one compound of formula (II) 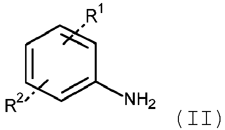 in which
R¹ and R² have the definitions specified in Claim 1, is reacted with a diazotization reagent, and the reaction mixture obtained is subsequently reacted with at least one compound of formula (III) 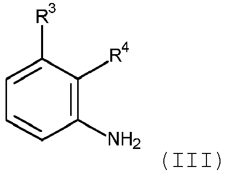 in which
R³ and R⁴ have the definitions specified in Claim 1,
to give the intermediate of formula (IV) 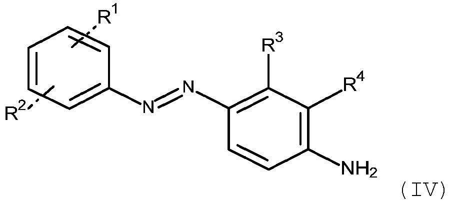 in which
R¹, R², R³ and R⁴ have the definitions specified in Claim 1,
and the intermediate of formula (IV) is subsequently reacted with a diazotization reagent, and then the reaction mixture thus obtained is reacted with a compound of formula (V) 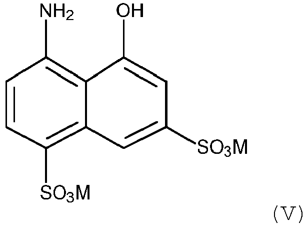
to give the intermediate of formula (VI) 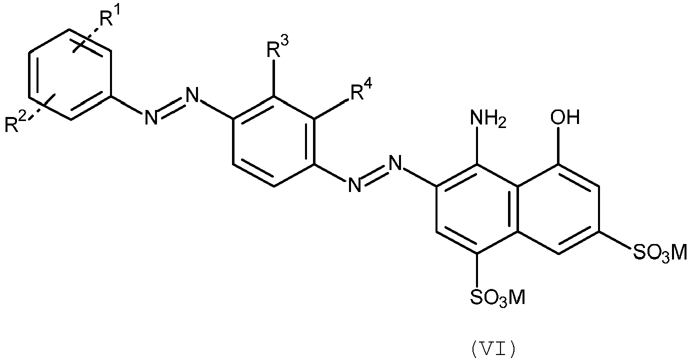 in which
R¹, R², R³ and R⁴ have the definitions specified in Claim 1,
and subsequently the intermediate of formula (VI) is further reacted with the reaction product obtainable by reacting a compound of formula (VII) 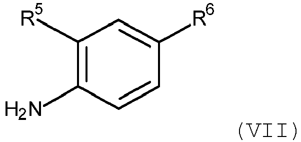 in which
R⁵ and R⁶ have the definitions specified in Claim 1, with a diazotization reagent, and the trisazo compound of formula (I) that is formed is subsequently isolated by filtration of the reaction mixture.

## Revendications

1. Composés trisazo de formule (I) 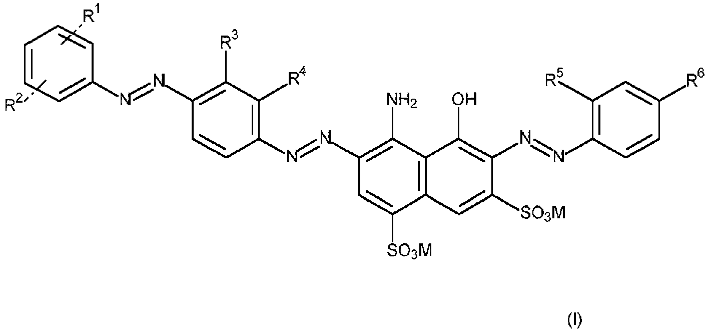 dans laquelle
R¹ représente SO₃M ou COOM et
R² représente SO₃M ou COOM,
R³ et R⁴ représentent, indépendamment l'un de l'autre, hydrogène ou CH₃,
R⁵ et R⁶ représentent, indépendamment l'un de l'autre, SO₃M, COOM ou NO₂ et
M représente hydrogène, un cation métallique monovalent, ammonium ou alkylammonium, qui est monosubstitué ou polysubstitué, de manière identique ou différente, par C₁-C₄-alkyle.

2. Composés trisazo selon la revendication 1, **caractérisés en ce que**
R¹ représente SO₃M ou COOM,
R² représente SO₃M ou COOM,
R³ et R⁴ représentent, indépendamment l'un de l'autre, hydrogène ou CH₃ et
R⁵ et R⁶ représentent, indépendamment l'un de l'autre, SO₃M, COOM ou NO₂,
étant entendu que l'un des radicaux R⁵ ou R⁶ représente NOz et
M représente hydrogène, sodium, potassium, lithium, ammonium ou alkylammonium, qui est monosubstitué ou polysubstitué, de manière identique ou différente, par C₁-C₂-alkyle.

3. Composés trisazo selon la revendication 1 ou 2, **caractérisés en ce que**
R¹ représente SO₃M ou COOM,
R² représente SO₃M ou COOM,
R³ et R⁴ représentent, indépendamment l'un de l'autre, hydrogène ou CH₃ et
R⁵ et R⁶ représentent, indépendamment l'un de l'autre, SO₃M, COOM ou NOz,
étant entendu que l'un des radicaux R⁵ ou R⁶ représente NOz et
M représente hydrogène, sodium, potassium, lithium, ammonium, triméthylammonium ou triéthylammonium.

4. Composés trisazo selon l'une des revendications 1 à 3, **caractérisés en ce que**
R¹ représente SO₃M ou COOM,
R² représente SO₃M ou COOM,
R³ et R⁴ représentent hydrogène ou CH₃,
étant entendu que R³ et R⁴ ne représentent pas tous les deux simultanément hydrogène et ne représentent pas tous les deux simultanément CH₃,
R⁵ et R⁶ représentent SO₃M, COOM ou NO₂,
étant entendu que l'un des radicaux R⁵ ou R⁶ représente NO₂ et
M représente hydrogène, sodium, potassium, lithium, ammonium, triméthylammonium ou triéthylammonium.

5. Composés trisazo selon l'une des revendications 1 à 4, **caractérisés en ce qu'**ils correspondent aux formules (Ia) ou (Ib) 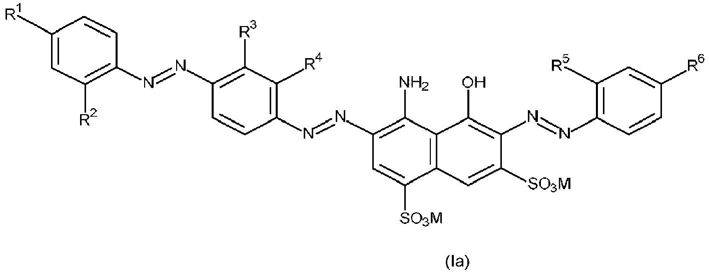 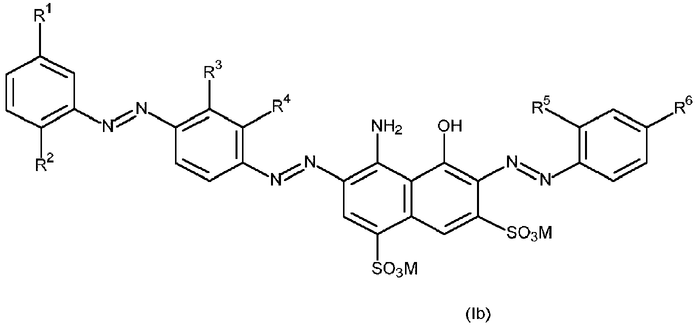 dans lesquelles
R¹ représente SO₃M ou COOM,
R² représente SO₃M ou COOM,
R³ et R⁴ représentent hydrogène ou CH₃,
étant entendu que R³ et R⁴ ne représentent pas tous les deux simultanément hydrogène et ne représentent pas tous les deux simultanément CH₃,
R⁵ et R⁶ représentent SO₃M, COOM ou NO₂,
étant entendu que l'un des radicaux R⁵ ou R⁶ représente NOz et
M représente hydrogène, sodium, potassium, lithium, ammonium, triméthylammonium ou triéthylammonium.

6. Composés trisazo selon l'une des revendications 1 à 5, **caractérisés en ce que**
R¹ représente SO₃M,
R² représente SO₃M,
R³ et R⁴ représentent hydrogène ou CH₃,
étant entendu que R³ et R⁴ ne représentent pas tous les deux simultanément hydrogène et ne représentent pas tous les deux simultanément CH₃,
R⁵ et R⁶ représentent SO₃M, COOM ou NO₂,
étant entendu que l'un des radicaux R⁵ ou R⁶ représente NOz et
M représente hydrogène, sodium, potassium, lithium, ammonium ou triéthylammonium.

7. Composés trisazo selon l'une des revendications 1 à 6, **caractérisés en ce que**
R¹ représente COOM,
R² représente COOM,
R³ et R⁴ représentent hydrogène ou CH₃,
étant entendu que R³ et R⁴ ne représentent pas tous les deux simultanément hydrogène et ne représentent pas tous les deux simultanément CH₃,
R⁵ et R⁶ représentent SO₃M, COOM ou NO₂,
étant entendu que l'un des radicaux R⁵ ou R⁶ représente NO₂ et
M représente hydrogène, sodium, potassium, lithium,
ammonium ou triéthylammonium.

8. Composés trisazo selon l'une des revendications 1 à 7, **caractérisés en ce qu'**ils correspondent aux formules 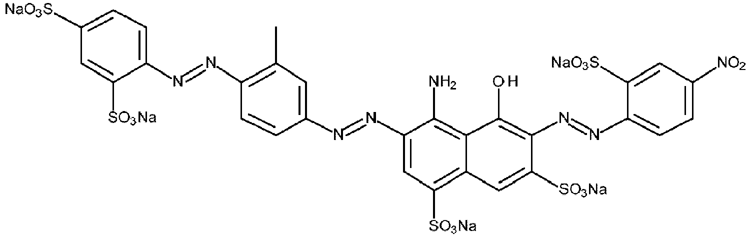 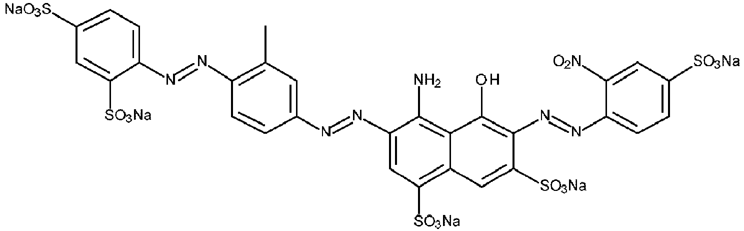 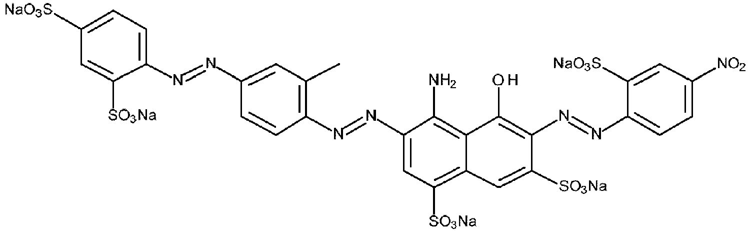 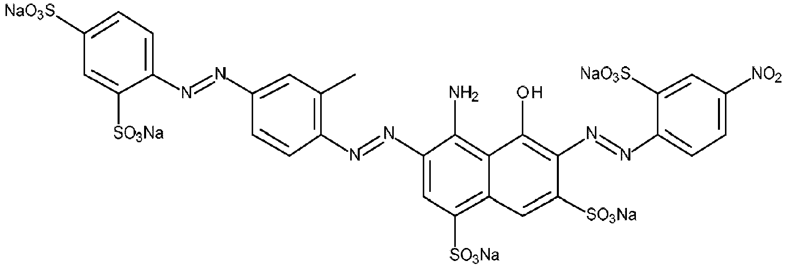 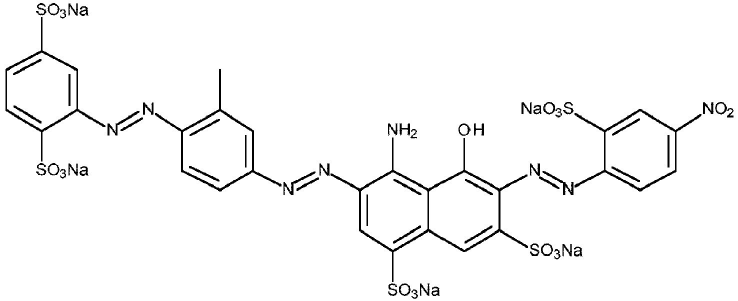 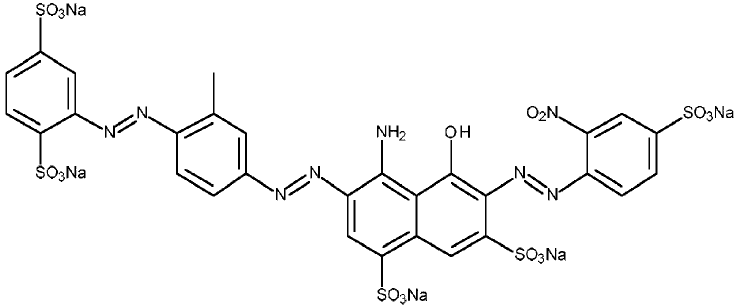 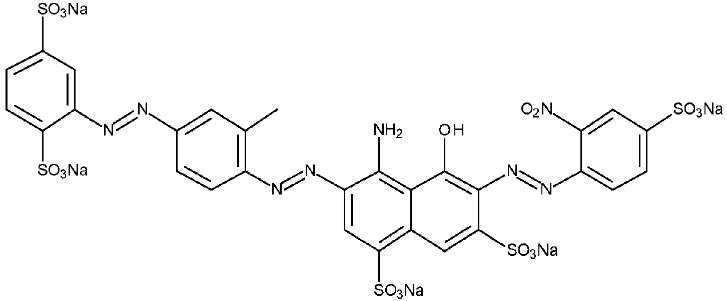 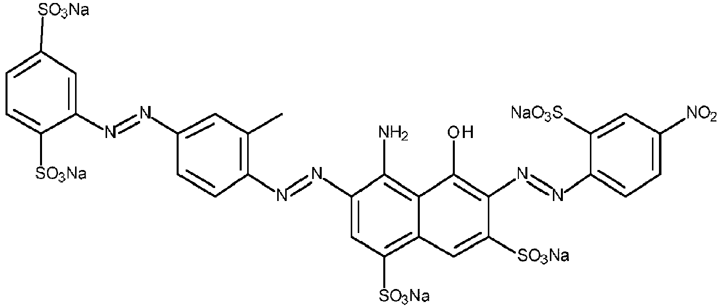 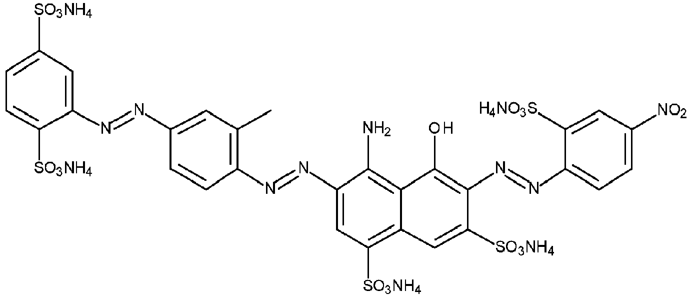 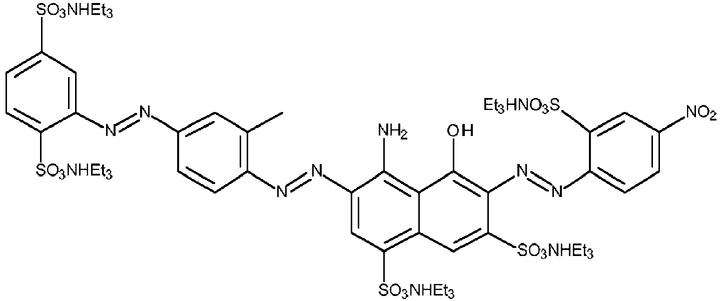 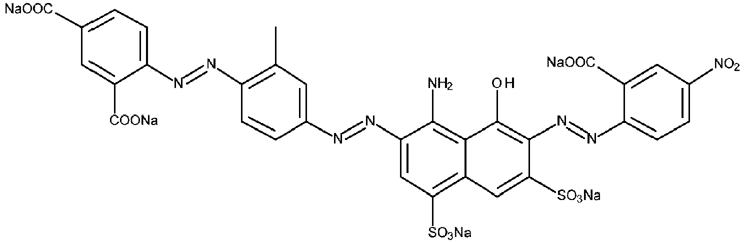 ou

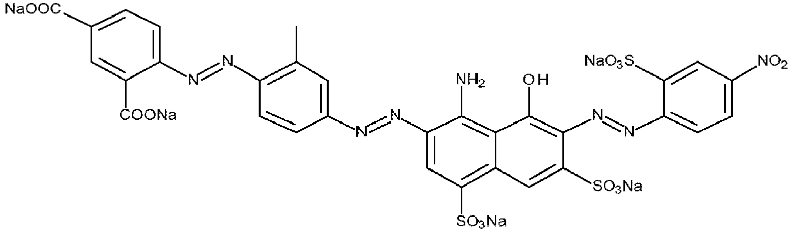

9. Procédé de coloration et d'impression de matériaux cellulosiques, de peaux animales, de poils d'animaux, de coquilles d'oeuf, de matériaux poreux et de métaux, **caractérisé en ce qu'**on utilise comme colorant au moins un composé trisazo selon l'une des revendications 1 à 8.

10. Formulation contenant au moins un composé trisazo selon l'une des revendications 1 à 8.

11. Formulation selon la revendication 10, **caractérisée en ce qu'**il s'agit d'une formulation liquide.

12. Formulation selon la revendication 10 ou 11, **caractérisée en ce qu'**elle contient 0,5 à 25% en poids, de préférence 1,0 à 15% en poids et de manière particulièrement préférée 2,0 à 8,0% en poids d'au moins un composé trisazo selon l'une des revendications 1 à 8, à chaque fois par rapport à la totalité de la formulation.

13. Formulation selon l'une des revendications 10 à 12, **caractérisée en ce qu'**elle contient 40 à 99% en poids, de préférence 70 à 95% en poids d'eau et le cas échéant un ou plusieurs additifs de la série N-méthyl-2-pyrrolidone, 2-pyrrolidone, 2-hexylpyrrolidone, hydroxyéthylpyrrolidone, 2-propanol, éthanediol, 1,2-hexanediol, 1,2-butanediol, triméthylolpropane, diéthylèneglycol, diéthylèneglycolmonobutyléther, triéthylèneglycolmonobutyléther, dipropylèneglycolmonobutyléther, glycérol, lactate de butyle, urée, sulfolane, glycoléther et biocides en une quantité totale de 0,01 à 50% en poids, de préférence de 0,1 à 20% en poids par rapport à la totalité de la formulation.

14. Formulation selon l'une des revendications 10 à 13, **caractérisée en ce qu'**elle contient 40 à 99% en poids, de préférence 70 à 95% en poids d'au moins un solvant de la série N-méthyl-2-pyrrolidone, 2-pyrrolidone, 2-hexylpyrrolidone, hydroxyéthylpyrrolidone, 2-propanol, éthanediol, 1,2-hexanediol, 1,2-butanediol, triméthylolpropane, diéthylèneglycol, diéthylèneglycolmonobutyléther, triéthylèneglycolmonobutyléther, dipropylèneglycolmonobutyléther, glycérol, lactate de butyle et le cas échéant un ou plusieurs additifs de la série urée, sulfolane, glycoléther, biocides en une quantité totale de 0,01 à 50% en poids, de préférence de 0,1 à 20% en poids par rapport à la totalité de la formulation.

15. Utilisation d'une formulation selon l'une des revendications 10 à 14 comme liquide d'enregistrement pour l'impression par jet d'encre, comme agent colorant dans des dispositifs d'écriture ou lors de la fabrication de filtres couleur pour des applications optiques et optoélectroniques.

16. Procédé de préparation de composés trisazo selon l'une des revendications 1 à 8, **caractérisé en ce qu'**au moins un composé de formule (II) 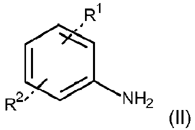 dans laquelle
R¹ et R² présentent les significations indiquées dans la revendication 1,
est transformé avec un réactif de diazotation et le mélange réactionnel obtenu est ensuite transformé avec un composé de formule (III) 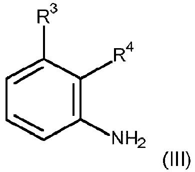 dans laquelle
R³ et R⁴ présentent les significations indiquées dans la revendication 1,
en produit intermédiaire de formule (IV) 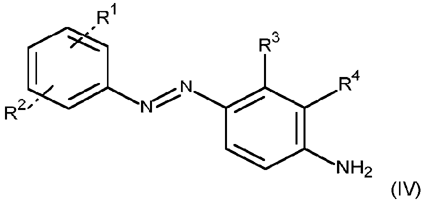 dans laquelle
R¹, R², R³ et R⁴ présentent les significations indiquées dans la revendication 1,
et le produit intermédiaire de formule (IV) est ensuite transformé avec un réactif de diazotation et le mélange réactionnel ainsi obtenu est ensuite transformé avec un composé de formule (V) 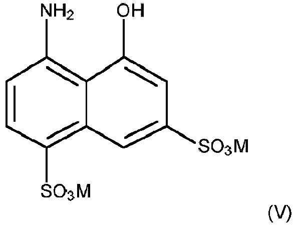 en produit intermédiaire de formule (VI) 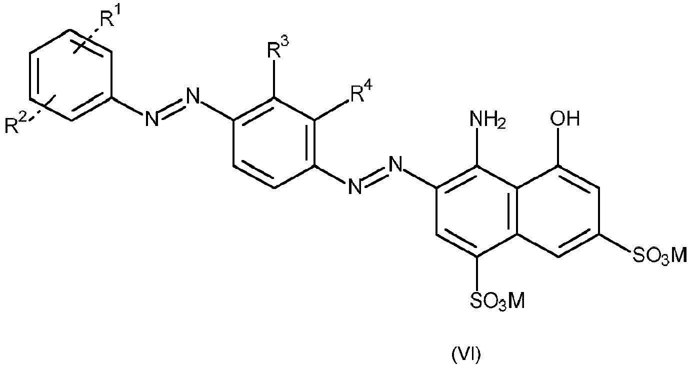 dans laquelle
R¹, R², R³ et R⁴ présentent les significations indiquées dans la revendication 1,
et le produit intermédiaire de formule (VI) est ensuite transformé davantage avec le produit de transformation qui peut être obtenu par transformation d'un composé de formule (VII) 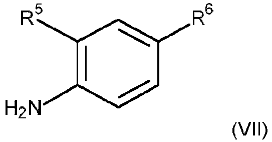 dans laquelle
R⁵ et R⁶ présentent les significations indiquées dans la revendication 1,
avec un réactif de diazotation et le composé trisazo de formule (I) formé est ensuite isolé par filtration du mélange réactionnel.
